# FASCICULE DE BREVET EUROPEEN

(11) **EP 1 328 506 B1**
(45) Date de publication et mention de la délivrance du brevet: **29.12.2004**
(21) Numéro de dépôt: 01983636.0
(22) Date de dépôt: 26.10.2001
(51) Int. Cl.: C07C 229/30, C07C 227/16, C07C 67/313, C07C 403/16

(54) **INTERMEDIAIRES UTILES POUR LA SYNTHESE DE RETINOIDES**
ZWISCHENPRODUKTE FÜR DIE HERSTELLUNG VON RETINOIDEN
INTERMEDIATES FOR USE IN RETINOID SYNTHESIS

(30) Priorité: 26.10.2000 FR 0013726
(43) Date de publication de la demande: 23.07.2003
(73) Titulaire: CENTRE NATIONAL DE LA RECHERCHE SCIENTIFIQUE (CNRS), 75794 Paris Cedex 16 (FR)
(72) Inventeur: VALLA, Alain, F-29750 Loctudy (FR); CARTIER, Dominique, F-29000 Quimper (FR); LABIA, Roger, F-29180 Plougonnec (FR); POTIER, Pierre, Jean-Paul, F-75007 Paris (FR)
(74) Mandataire: Warcoin, Jacques
(86) Numéro de dépôt international: PCT/FR2001/003331
(87) Numéro de publication internationale: WO 2002/034710

(56) Documents cités:
- EP-A- 0 802 180
- DE-A- 2 816 226
- DE-B- 1 046 612
- DE-B- 1 059 900
- FR-A- 1 055 849
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-Accession no. 4248648 (BRN), XP002170672 & BULL. ACAD. SCI. USSR DIV. CHEM. SCI. (ENGL. TRANSL.), vol. 39, no. 2.1, 1990, pages 298-306,
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-Accession no. 2107391 et 2095388 (BRN), XP002170673 & BULL. ACAD. SCI. USSR DIV. CHEM. SCI. (ENGL. TRANSL.), vol. 24, 1975, pages 2397-2401,
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-Accession no. 4425660 (BRN), XP002170674 & CHEM. HETEROCYCL. COMPD. (ENGL. TRANSL.), vol. 24, no. 10, 1988, pages 1095-1103,
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-Accession no. 4393860 et 4410277 (BRN), XP002170675 & CHEM. HETEROCYCL. COMPD. (ENGL. TRANSL.), vol. 24, no. 10, 1988, pages 1095-1103,
- DATABASE CROSSFIRE BELSTEIN [en ligne] BEILSTEIN INSTITUT ZUR FOEDERUNG DER CHEMISCHEN WISSENSCHAFTEN, FRANKFURT AM MAIN, DE; Database-Accession no. 6805029 (BRN), XP002170676 & JOURNAL OF THE AMERICAN CHEMICAL SOCIETY., vol. 116, no. 6, 1994, pages 2619-2620, DC US
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-Accession no. 6323248 (BRN), XP002170677 & JOURNAL OF THE AMERICAN CHEMICAL SOCIETY., vol. 115, no. 7, 1993, pages 3006-3007, DC US
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-Accession no. 2081265 (BRN), XP002170678 & BER. BUNSEN-GES. PHYS. CHEM., vol. 80, 1976, pages 630-636,
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-Accession no. 8320158 (BRN), XP002170679 & PHARMAZIE, vol. 54, no. 8, 1999, pages 571-574,
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-accession no. 8499410 (BRN), XP002170680 & JOURNAL OF ORGANIC CHEMISTRY., vol. 64, no. 26, 1999, pages 9493-9498, EASTON US
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-Accession no. 2366899 et 2368323 (BRN), XP002170681 & BULL. ACAD. SCI. USSR DIV. CHEM. SCI. (ENGL. TRANSL.), vol. 22, 1973, pages 2478-2482,
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-Accession no. 1951457 (BRN), XP002170682 & BULL. ACAD. SCI. USSR DIV. CHEM. SCI. (ENGL. TRANSL.), vol. 22, 1973, pages 1963-1971,
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-Accession no. 1962096 (BRN), XP002170683 & IVZ. AKAD. SSSR, 1972, pages 2153-2218,
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-accession no. 6056003 (BRN), XP002170684 & BULL. ACAD. SCI. USSR DIV. CHEM. SCI. (ENGL. TRANSL.), vol. 29, 1980, pages 1643-1651,
- DATABASE CROSSFIRE BEILSTEIN [en ligne] BEILSTEIN INSTITUT ZUR FOEDERUNG DER CHEMISCHEN WISSENSCHAFTEN, FRANKFURT AM MAIN, DE; Database-Accession no. 6053910 (BRN), XP002170685 & BULL. ACAD. SCI. USSR DIV. SCI. (ENGL. TRANSL.), vol. 30, no. 2, 1981, pages 308-311,

## Description

La présente invention a pour objet de nouveaux intermédiaires utiles pour la synthèse de rétinoïdes, de caroténoïdes, et leur préparation. Elle concerne aussi un nouveau procédé de synthèse de rétinoïdes, notamment de l'acide rétinoïque et de la vitamine A ou rétinol via l'acide rétinoïque ainsi obtenu.

Les rétinoïdes, notamment la vitamine A, sont utilisés dans des domaines variés, notamment en thérapeutique, en cosmétologie et en agro-alimentaire et de nombreux procédés de synthèse ont été utilisés.

De nombreux procédés de préparation ont été mis en oeuvre pour la synthèse des rétinoïdes, notamment pour celle de l'acide rétinoïque et de la vitamine A et un article récent relatif aux techniques industrielles permettant d'accéder à la vitamine A a été écrit par Paust J. (Pure & *Appl. Chem*. (1991), **63**, 45-58).

La première synthèse de rétinoïdes appartenant à la famille de la vitamine A a été décrite par R. Kuhn et C.J.O.R. Morris (Ber. (1937), **70**, 853).

La condensation de β-ionylidèneacétaldéhydes avec le 3-méthylcrotonate d'éthyle conduit à différents acides rétinoïques, suivant la configuration de l'aldéhyde, la nature du solvant et la base utilisée pour la génération de l'anion (M. Matsui, S. Okano, K. Yamashita, M. Miyano, S. Kitamura, A. Kobayashi, T. Sato et B.R. Mikami *J. Vitaminol*. (Kyoto), (1958), **4**, 178).

Ces mêmes isomères ont été synthétisés par une voie similaire à partir de différents aldéhydes en C-15 et du β-méthylglutaconate de méthyle (C.D. Robeson, J.D. Cawley, L. Weisler, M.H. Stern, C.C. Eddinger et A.J. Chechak, *J. Am. Chem. Soc*. (1955), **77**, 4111).

La synthèse de l'acide 13*Z*-rétinoïque a été réalisée également par une réaction de Réformatskii entre l'aldéhyde en C-15 et le γ-bromo-β-méthylcrotonate de méthyle (Eiter, E. Truscheit et H. Oediger, *Angew. Chem*. (1960), **72**, 948).

La réduction des différents esters méthyliques en C-20, par LiAlH₄, conduit aux isomères correspondants de la vitamine A. Le rétinal est obtenu à partir de cette dernière par oxydation (brevet US 3 367 985; J.D. Surmatis, S. Ball, T.W. Goodwin et R.A. Morton, *Biochem. J*. (1948), **42**, 516; C.D. Robertson, W.P. Blum, J.M. Dieterle, J.D. Cawley et J.G. Baxter *J. Am. Chem. Soc*. (1955), **77**, 4120).

D'une façon similaire, les dérivés de l'α-vitamine A ont été préparés par condensation du 3-méthylcrotonate d'éthyle avec l'α-ionylidèneacétaldéhyde (P.S. Marchand, R. Rüegg, U. Schwieter, P.T. Siddons et B.C.L. Weddon, J. *Chem. Soc*.(1965), 2019).

Les réactions de Wittig et Horner, à partir de l'aldéhyde en C-15, ont été largement utilisées pour la synthèse de nombreux dérivés de la vitamine A.

Ainsi, les esters de l'acide rétinoïque ont été préparés en utilisant:
- le sel de phosphonium issu du γ-bromo-β-méthylcrotonate de méthyle (brevet DE 950 552; G. Wittig et H. Pommer, *Chem. Abstr*. (1959), **53**, 436), et
- le phosphonate équivalent (H. Pommer, *Angew*.*Chem*. (1960), **72**, 811).

Le rétinonitrile et l'acétate de vitamine A ont été préparés en utilisant:
- le sel de phosphonium issu du γ-bromo-β-crotonitrile (H. Pommer, *Angew. Chem*. (1960), **72**, 811), et
- le phosphonate correspondant (brevet DE 1 116 652; H. Pommer et W. Stilz, *Chem. Abstr*. (1962), **57**, 2267).

Le rétinal a été également synthétisé par:
- une réaction de Wittig avec le bromure de (4,4-diéthoxy-2-méthyl-2-butényl)triphénylphosphonium,
- condensation de l'acétal diéthylique du β-ionylidèneacétaldéhyde avec le 1-éthoxy-3-méthyl-1,3-butadiène (S. M. Makin, *Russ. Chem. Rev*. (Engl.Transl.) (1969), **38**, 237), et
- oxydation selon Oppenauer d'un mélange de β-ionylidèneéthanol et d'alcool β, β-diméthylallylique (brevet JP 5145/58; M. Matsui et S. Kitamura, *Chem. Abstr*. (1959), **53**, 17,178).

Le procédé développé par H. Pommer (*Angew*. *Chem*. (1960), **72**, 811), actuellement utilisé par la société Badische Anilin-& Soda Fabrik AG (BASF) met en jeu une réaction de Wittig entre le chlorure de (β-ionylidèneéthyl) triphénylphosphonium et le γ-acétoxytiglaldéhyde, en présence de méthylate de sodium. Le produit réactionnel est constitué d'un mélange d'acétates *tout E* et 11 *Z* à partir duquel l'isomère tout E est obtenu par cristallisation (brevet DE 1 058 710; H. Pommer et W. Sarnecki, Chem. Abstr. (1961), **55**, 12, 446; brevet DE 1 046 612; H. Pommer et W. Sarnecki, Chem. Abstr. (1961), **55**, 5573 et brevet DE 1 059 900; H. Pommer et W. Sarnecki, *Chem. Abstr*. (1961), **55**, 14, 511).

L'acide rétinoïque et l'ester éthylique correspondant ont été synthétisés par la même voie en substituant respectivement l'acide β-formylcrotonique ou le β-formylcrotonate d'éthyle au γ-acétoxytiglaldéhyde (brevet DE 1 058 710; H. Pommer et W. Sarnecki, Chem. Abstr. (1961), **55**, 12,446 ; brevet DE 1 046612; H. Pommer et W. Sarnecki, *Chem. Abstr*. (1961), **55**, 5573; brevet DE 1 059 900; H. Pommer et W. Sarnecki, *Chem. Abstr*. (1961), **55**, 14, 511; brevet DE 1 068 702; H. Pommer et W. Sarnecki, *Chem. Abstr*. (1961), **55**, 10,812).

L'acide 9*Z*-rétinoïque a été préparé à partir du sel de phosphonium en C-15 issu du *β-*(*Z*) ionylidèneéthanol (brevet DE 1 068 710; H. Pommer et W. Sarnecki, *Chem. Abstr*. (1961), **55**, 12, 446). Ainsi, 1a synthèse de l'acide rétinoïque (et de ses esters) utilisée industriellement par BASF met en jeu une réaction de Wittig entre le phosphorane et un aldéhyde en C-5 (H. Pommer et W. Sarnecki, déjà cités), ledit phosphorane étant synthétisé par l'action de la triphénylphosphine sur le β-ionol.

La première synthèse stéréosélective de l'acide 11*Z*,13*Z*-rétinoïque a été réalisée en 1965 par Pattenden *et al*., par réaction de Wittig entre le phosphorane (généré à partir du bromure de β-ionylidène-éthyltriphényl-phosphonium) et le 4-hydroxy-3-méthyl-2-buten-4-olide (G. Pattenden, B.C.L. Weedon, C.F. Garbers, D.F. Schneider et J.P. van der Merwe, Chem. Commun. (1965) 347). Le mélange réactionnel évolue en conduisant à un mélange d'acides rétinoïques que l'on peut séparer par cristallisation fractionnée. L'isomérisation contrôlée de l'acide 11*Z*,13*Z*-rétinoïque conduit respectivement aux acides 13*Z* ou *all E* rétinoïques (G. Pattenden et B.C.L. Weedon, *J. Chem. Soc. C* (1968), 1984 ; C.F. Garbers, D.F. Schneider et J.P. van der Merwe, *J. Chem. Soc*. *C* (1968), 1982).

Une approche similaire, décrite par G. Cainelli et G. Cardillo (*Acc*. *Chem. Res*. (1981), **14**, 89) et G. Cainelli, G. Cardillo, M. Contendo, P. Grasselli et A. Umani Ronchi (*Gazz. Chim. Ital*. (1973), **103**, 117) met en oeuvre la réaction du diénolate (issu du sel de sodium de l'acide sénécioïque) sur l'aldéhyde en C-15.

Alternativement, R.W. Dugger et C.H. Heathcock (*J. Org. Chem*., (1980), **45**, 1181) ont préparé des lactones par action du diénolate (dérivé du sénécioate d'éthyle) sur le *E* et le *Z* β-ionylidèneacétaldéhyde.

G. Cardillo, M. Contendo et S. Sandri, (*J*.*Chem*.*Soc*. *Perkin* I (1979), 1729) ont proposé une synthèse stéréospécifique du *all trans* rétinal *via* le β-ionylidèneacétaldéhyde et le dianion dérivé du 3-méthyl-3-buténol.

La préparation de la vitamine A et celle de l'ester éthylique de l'acide rétinoïque ont été également réalisées par condensation du β-formylcrotonate de méthyle sur les dérivés de Grignard en C-15 (K.K. Chugai Seiyaku *Derwent Farmdoc* (1964), **11**, 643; K. Shishido, H. Nozaki et M. Tsuda, *Derwent Farmdoc*, (1964), **11**, 669).

La condensation de l'anion du bis(2,2,2-trifluoroéthyl)-phosphonate (généré par l'hexaméthyldisilazide de potassium/18-crown-6) sur le 7*Z*,9*Z*-β-ionylidène-acétaldéhyde conduit au mélange des 7*Z*,9*Z*-, 7*Z*,9*Z*,11*Z*-, 7*Z*,9*Z*,13*Z*- et 7*Z*,9*Z*,11*Z*,13*Z*-rétinonitriles, qui peuvent être séparés par chromatographie liquide haute performance (HPLC) (A. Trehan, T. Mirzadegan et R.S.H. Liu, *Tetrahedron*, (1990), **46**, 3769 ; W.C. Still et C. Gennari, *Tetrahedron Lett*. (1983), **24**, 4405).

D'autres auteurs ont utilisé l'ester benzoïque du 7Z-éthynylcarbinol pour préparer le 11*Z*-18,14-rétrorétinol. L'isomérisation photochimique de l'éthynyl-β-ionol a permis d'obtenir le composé possédant une configuration *Z* en C-7 (Y.S. Chauhan, R.A.S. Chandraratna, D.A. Miller, R.W. Kondrat, W. Reischl et W.H. Okamura, *J. Am. Chem. Soc*. (1985), **107**, 1028). L'hydrolyse du benzoate par une base, comme par exemple le 1,8-diazabicyclo[5.4.0]undec-7-ène (DBU), permet la formation du système rétro.

La condensation de l'aldéhyde en C-15 de configuration *E*, avec l'isopropylidène malonate diéthylique suivie de l'hydrolyse de l'ester, a conduit à l'acide 14-carboxy-rétinoïque (Y.F. Shealy, C.A. Krauth, J.M. Riodan et B.P. Sani, *J. Med. Chem*. (1988), **31**, 1124).

La condensation du β-ionylidèneacétaldéhyde, diméthylacétal avec le triméthylsilyldiénol éther ou l'éthyl-diénol éther, catalysée par un acide de Lewis, conduit au mélange de 11-méthoxy- et de 11,12-dihydrorétinals qui, par élimination de méthanol sous l'action d'une base suivie d'une isomérisation (I₂), produit le *all trans* rétinal (T. Mukaiyama et I. Ishida *Chem. Lett*. (1975), 1201).

M. Julia et D. Arnould (*Bull. Soc. Chim. Fr*. (1973), 746) ont rapporté une synthèse stéréosélective dans laquelle la chaîne tétraénique est générée par une alkylation *via* un acide sulfinique, suivie d'une élimination dudit acide.

Cette synthèse a été reprise par les sociétés Hoffmann-La Roche (P.S. Manchand, M. Rosenberg, G. Saucy, P.A. Wehrli, H. Wong, L. Chambers, M.P. Ferro et W. Jackson, *Helv*. *Chim. Acta* (1976), **59**, 387) et Rhône Poulenc Industries (P. Chabardes, J.P. Decor et J. Varagnat, *Tetrahedron Lett*. (1977), **33**, 2799) pour la production de vitamine A sous la forme *all trans* et de l'acétate correspondant (pour ce dernier, en partant de C-5 chloroacétate). Les stéréosélectivités étant de 9*E*:9*Z* ≃ 73:27 à 82:18.

Olson et al. ont décrit une préparation de all trans vitamine A (sous forme d'acétate), en partant de *all trans* C-15 bromotriène, préparé à partir de vinyl-β-ionol, et de tolylsulfone en C-5 (G.L. Olson, H.C. Cheung, K.D. Morgan, C. Neukom et G. Saucy *J. Org. Chem*. (1976), **41**, 3287).

Manchand *et al*. rapportent une séquence utilisant une autre unité en C-5, avec la sulfone en C-15, pour la synthèse de l'acétate de rétinyle. Le complexe π d'allylpalladium/prénylacétate est alkylé de manière stéréosélective en position δ par l'anion de la sulfone (P.S. Manchand, H.S. Wong et J.F. Blount *J. Org. Chem*. (1978), **43**, 4769-74).

Par photoisomérisation de ces composés, on peut obtenir les isomères 7*Z*;7*Z*, 11*Z*;7*Z*, 13*Z*;7*Z*,9*Z*,11*Z et all Z* (R.S.H. Liu et A.E. Asato *Tetrahedron Lett*. (1984), **40**, 1931).

Une synthèse récente permet également de préparer les acides 13*Z* et 13*E* rétinoïques par l'emploi de nouveaux synthons β-méthylène aldéhydes, par condensation selon Stobbe avec l'isopropylidène malonate de méthyle, en présence de triton B (demande de brevet EP 0 802 180; Giraud M. *et al*.).

A l'heure actuelle, les trois principales synthèses industrielles utilisent comme produit de départ la β-ionone.

La stratégie d'Hoffmann-La Roche dérive de la technique d'Isler (O. Isler, W. Huber, A. Ronco et M. Kofler *Helv. Chim. Acta* (1947), **30**, 1911 ; O. Isler, A. Ronco, W. Guex, N.C. Hindley, W. Huber, K. Dialer et M. Kofler, *Helv. Chim. Acta* (1949), **32**, 489) et met en oeuvre un synthon en C-14 et un synthon en C-6.

Le synthon en C-6 utilisé est lui-même préparé en 3 étapes.

Une autre synthèse industrielle développée par Badische Anilin-& Soda Fabrik AG (BASF) est basée sur la méthode de Inhoffen et Pommer (H. Pommer *Angew*. *Chem*.(1960), **72**, 811).

Le synthon utilisé pour l'élongation de la chaîne est préparé en 4 étapes.

La synthèse de la vitamine A réalisée par Rhône Poulenc Rorer (RPR) est adaptée de la technique de Julia (Julia M. et Arnould D. *Bull. Soc. Chim*. (1973), 746).

Dans ce procédé, on utilise un synthon en C-6 préparé en 4 étapes.

EP 802 180 concerne des β-méthylènealdéhyde utiles dans la préparation de dérivés de rétinoïdes ou de caroténoïdes. Ces nouveaux intermédiaires ouvrent la voie à un procédé de préparation de dérivés de rétinoïdes et de caroténoïdes avec d'excellents rendements et dans des conditions douces et évitent l'utilisation de bases fortes telles que l'amidure de sodium, habituellement employées dans les condensations avec des composés à hydrogène activé.

DE 10 46 612 décrit un procédé de préparation de la vitamine A et de ses dérivés caractérisé en ce que l'on fait réagir le 5-[2',6',6',-triméthylcyclohexèn-(2')-yliden-(1')]-3-méthylpentadièn-(1,3) avec de la triarylphosphine en présence d'un dérivé halogéné et d'un aldéhyde de formule :

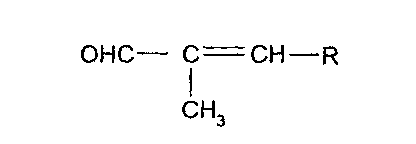

Ce procédé permet d'obtenir la vitamine A et ses dérivés d'une manière simple et comprend peu d'étapes.

DE 10 59 900 décrit un procédé de préparation de dérivés de vitamine A caractérisé en ce que l'on fait réagir le 5-[2',6',6',-triméthylcyclohexène-(1')-yl-(1')]-3-méthylpentadièn-(1,4)-ol-(3) (vinyl-β-ionol) avec un dérivé de triarylphosphine halogéné en présence d'un composé d'un aldéhyde de formule :

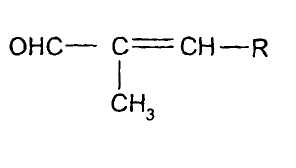

Ce procédé permet d'obtenir les dérivés de la vitamine A avec une très grande pureté sous forme cristalline.

FR 1 055 849 décrit un procédé de synthèse d'un dérivé de vitamine A, notamment à partir de la β-ionone par lequel on accroît la longueur de la chaîne latérale de la β-ionone par conversion en β-ionylidèneacétaldéhyde. Cet aldéhyde est condensé sur un ester de l'acide isopropylidènemalonique qui conduit à la formation d'un diacide de la vitamine A à partir duquel on fabrique le monoacide correspondant que l'on convertit en alcool vitaminique ou en ester de cet alcool.

Tous les procédés industriels rappelés ci-dessus utilisent des réactifs dangereux et/ou toxiques, notamment du sodium dans l'ammoniac liquide, des organométalliques, des hydrures d'aluminium, de l'oxychlorure de phosphore, du chlore et de la pyridine; ils nécessitent également la mise en oeuvre d'étapes d'hydrogénation catalytique qui sont particulièrement dangereuses. La préparation des synthons comprend plusieurs étapes et tous ces procédés requièrent en outre la mise en oeuvre d'étapes de purification pour éliminer les produits secondaires, ce qui rend ces procédés longs et coûteux.

Compte-tenu de l'importance des rétinoïdes et de leurs dérivés, notamment celle de l'acide rétinoïque et de la vitamine A, il est indispensable de trouver des procédés de synthèse qui pallient ou limitent ces inconvénients.

Aussi, les inventeurs se sont donnés pour but de synthétiser des composés utiles comme intermédiaires dans la synthèse des rétinoïdes et de leurs dérivés, notamment dans celle de l'acide rétinoïque et de la vitamine A, qui soient faciles à mettre en oeuvre.

La présente invention a pour objet des composés de formule (1) 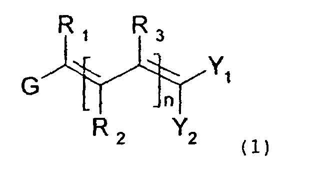 dans laquelle
G représente :
   - soit un groupe -NR₄R₅ où R₄ et R₅, identiques ou différents, sont chacun un groupe alkyle de 1 à 5 atomes de carbone, linéaire ou ramifié, ou un groupe cycloalkyle de 3 à 7 atomes de carbone, insaturé ou non, substitué ou non,
   ou R₄ et R₅ forment un cycle avec l'atome d'azote qui les porte,
R₁ représente :
   - soit un atome d'hydrogène,
   - soit un groupe alkyle de 1 à 5 atomes de carbone, linéaire ou ramifié,
   n est un nombre entier compris entre 1 et 2,
R₂ et R₃ représentent des substituants identiques ou différents dans chacun des motifs insaturés, lesdits motifs insaturés successifs pouvant être par ailleurs identiques ou différents, R₂ et R₃ étant choisis dans le groupe comprenant l'hydrogène, et les groupes alkyles de 1 à 5 atomes de carbone, linéaires ou ramifiés, **et** à condition qu'au moins un des substituants R₁, R₂ et R₃ soit différent de H, **et**
Y₁ et Y₂, représentent un groupe -COOR₇ où R₇ est un groupe alkyle de 1 à 5 atomes de carbone.

Au sens de la présente invention, on entend par groupe cycloalkyle, un cycle de 1 à 7 atomes de carbone, saturé ou comportant une ou deux insaturations, éventuellement substitué par un ou plusieurs groupements alkyles (de 1 à 4 atomes de carbone). A titre d'exemple non limitatif, on peut citer les groupes 2,6,6-triméthylcyclohexényle et 2,6,6-triméthylcyclohexadiényle.

Au sens de la présente invention, on entend par groupe protecteur de la fonction hydroxyle, des groupes tels que ceux définis par T. W. Greene dans "*Protective groups in organic synthesis*" (John Wiley Interscience, Ed. 2^{nd} Ed., 1991). A titre d'exemple non limitatif, on peut citer les éthers d'alkyles, les éthers silylés, les éthers phosphorés, les esters, les carbonates et les sulfonates.

Des composés de structure proche correspondant à la formule (1) pour laquelle n=0 sont connus et décrits dans la littérature.

Ainsi le dérivé monoéthylénique non substitué (1-a) 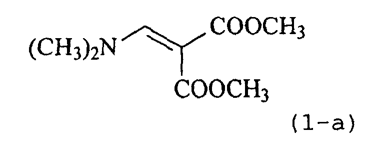 a été synthétisé par plusieurs auteurs notamment par Sorsak G., Grdadolnik S. et Stanovnik B. (*Ach. Mod. Chem*. (1998), **135**, 613-24) à partir du diméthylacétal du diméthylformamide (DMFDMA).

Le dérivé monoéthylénique non substitué (1-b) 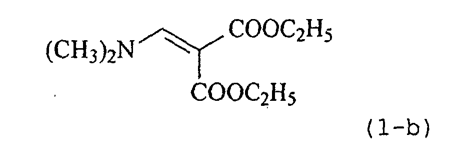 a été synthétisé par Regitz M. et Himbert G. (Justus *Liebigs Ann. Chem*. (1970), **734**, 70-85) et plus récemment par Gabbutt C., Hepworth J., Heron B., Elsegood M. et Clegg W. (*Chem. Commun*. (1999), **3**, 289-90).

Certains composés de formule (1) où n=1 sont également connus et décrits dans la littérature.

La synthèse du dérivé diéthylénique non substitué (1-c) a été décrite par Krasnaya Zh. *et al*. (*Bull. Acad. Sci. USSR Div. Chem. Sci., Engl. Transl*. (1973), **22**, 1959-62)

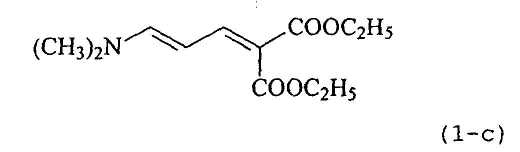

La synthèse de dérivés diéthyléniques substitués (1-d) où R et R' représentent notamment des groupes alkyles ou aryles, a été décrite par Smetskaya N.I., Mukhina N.A. et Granik V.G. (*Chem. Heterocycl. Compd*., Engl. Transl.(1984), **20**,650-53) et Michalik M., Zahn K., Köeckritz P. et Liebscher J. (*J. Prakt Chem*. (1989), **331**, 1-10; *Bull. Acad. Sci*. *USSR Div*. *Chem. Sci. Engl*. *Transl*. (1973), **22**, 1959-62)

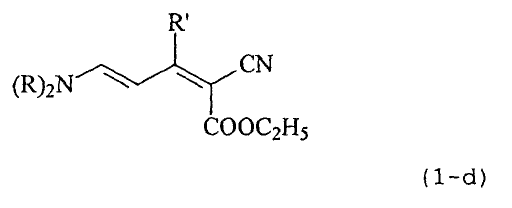

Ces mêmes auteurs décrivent également des composés (1-e) où R, R' et R" représentent notamment des groupes alkyles ou aryles.

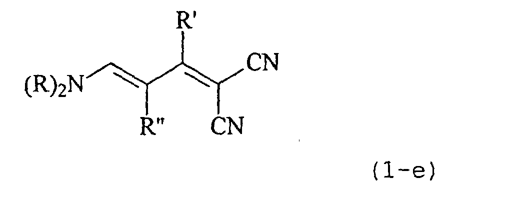

Toutefois les composés (1-d) et (1-e) n'ont jamais été utilisés dans la synthèse de rétinoïdes.

Le dérivé monoéthylénique non substitué (1-f) 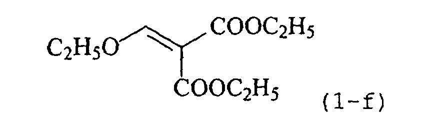 a été largement décrit et utilisé, notamment pour la synthèse des énamines correspondantes ou d'hétérocycles (Yamashkin S. et Yurovskaya M. *Chem. Heterocycl. Compd*., *Engl. Transl*. (1997), **33**, 1284-87 ; Glushkov R., Vozyakova T., Adamskaya E., Gus'kova T. *et al. Pharm. Chem. J., Engl. Transl*., (1998), **32**, 8-12 ; Kim Y., Kwon T., Chung S. et Smith M. *Synth. Commun*. (1999), **29**, 343-50).

Le dérivé diéthylénique non substitué (1-g) 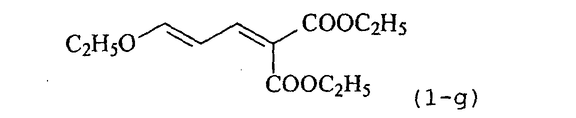 a été synthétisé notamment par Engel C. *et al. Can. J. Chem*. (1973), **51**, 3263-71 ; Overman L. et Robichaud A. *J. Am. Chem. Soc*. (1989), **111**, 300-308; Krasnaya Zh., Stytsenko T. et Bogdanov V. *Bull. Acad. Sci. USSR Div. Chem. Sci., Engl. Transl*. (1990), **39**, 2316-21 ; Gelin R. et Makula D. *Bull. Soc. Chim. Fr*. (1968), 1129-35 ; de Bie D., Geurtsen B., Berg I. et van der Henk C. *J. Org. Chem*. (1896), **51**, 3209-11.

La synthèse du dérivé diéthylénique non substitué (1-h) a également été décrite par Shramoya Z. *et al*. (*J. Org. Chem. USSR (Engl. Transl*.) (1966), **2**, 1005-1009) et par Gelin R. et Makula D. (*Bull. Soc. Chim. Fr. (*1968), 1129-35)

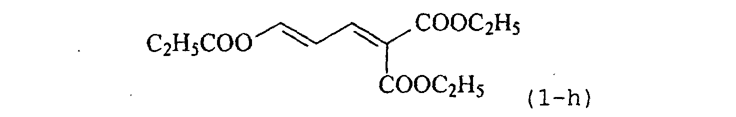

Le dérivé diméthylique (1-x) 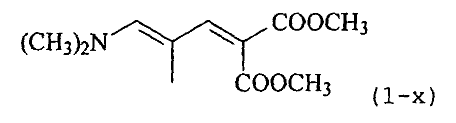 a été synthétisé par Bogdanov *et al*., *Bull. Acad. Sci. USSR Div. Chem. Sci*. (Engl. Transl.) (1990), **39**, 298-306 et 1172-80.

Toutefois, les composés (1-f) à (1-h) et (1-x) n'ont jamais été utilisés dans la synthèse de rétinoïdes.

Dans un mode particulier de réalisation, les composés de formule (1) sont ceux pour lesquels,
- G représente le groupe -N(CH₃)₂, -N(C₂H₅)₂ ou N-pyrrolidine,
- n est un nombre entier égal à 1 ou 2,
- R₁, R₂ et R₃ représentent, indépendamment l'un de l'autre, un atome d'hydrogène ou un groupe méthyle, les motifs insaturés successifs pouvant être par ailleurs identiques ou différents, à condition qu'au moins un des substituants R₁, R₂ et R₃ soit différent de H, et
- Y₁ et Y₂ représentent chacun un groupe -COOCH₃ ou un groupe -COOC₂H₅.

Dans un **autre** mode avantageux de réalisation selon l'invention, les composés sont choisis dans le groupe constitué par : 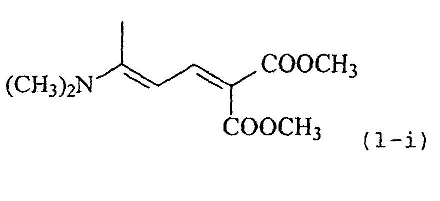 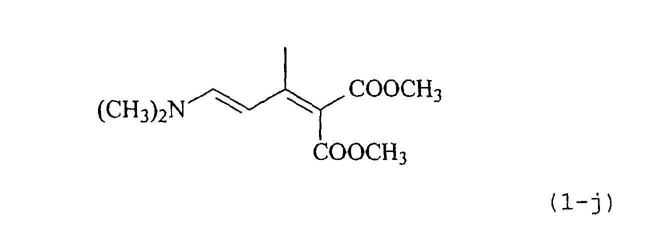 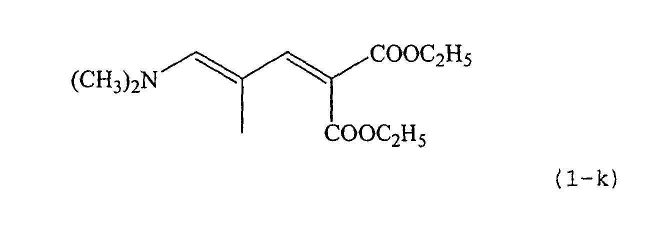 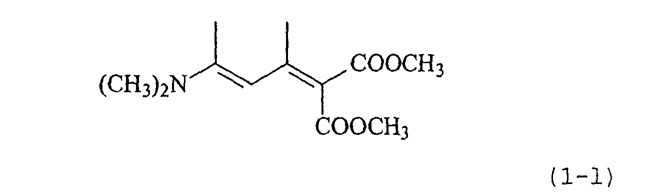 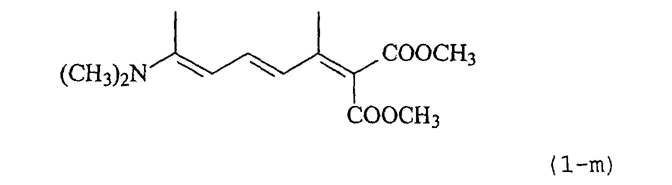 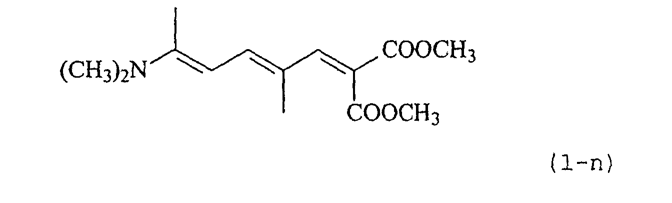 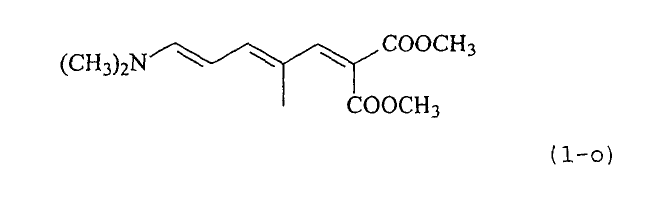 et les analogues esters éthyliques des composés (1-i) à (1-j) et (1-1) à (1-o).

Les composés selon l'invention peuvent être préparés par des techniques connues de l'homme du métier, à partir de produits de formule (3) 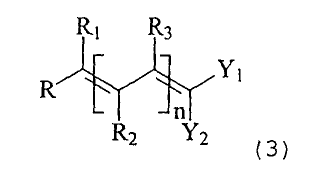 dans laquelle R₁, R₂ et R₃, Y₁ et Y₂ sont tels que définis précédemment et R représente un groupe alkyle (de 1 ou 2 atomes de carbone), disponibles dans le commerce ou qui peuvent être obtenus par des méthodes décrites dans la littérature, ou par le procédé décrit dans la présente demande.

Ainsi le composé (1-j) peut être préparé en faisant réagir l'isopropylidène malonate de méthyle de formule (3-a) (n=0, R=R₁=CH₃ et Y₁=Y₂= -COOCH₃). 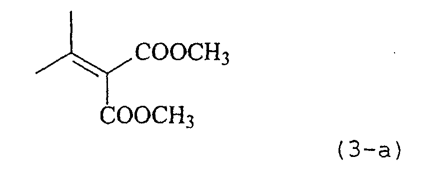 avec une forme protégée du *N,N*-diméthylformamide, par exemple le diméthylacétal, de formule (2-a) ou DMFDMA

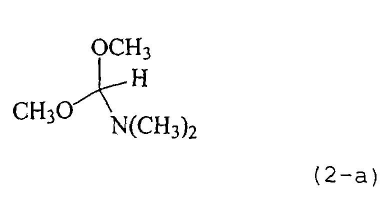

De même, les composés 1-i, 1-1, 1-m et 1-n, selon l'invention, peuvent être préparés à partir du diméthylacétal de la *N,N*-diméthylacétamide de formule (2-b)

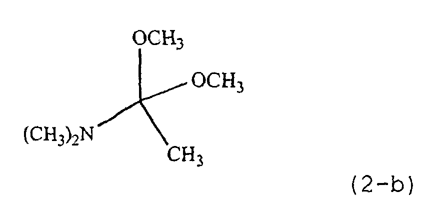

Les composés de formule (1), dans laquelle G représente un groupe -NR₄R₅ où R₄ et R₅ sont définis tels que précédemment, peuvent être obtenus d'une manière analogue à celles utilisées dans le schéma qui suit:

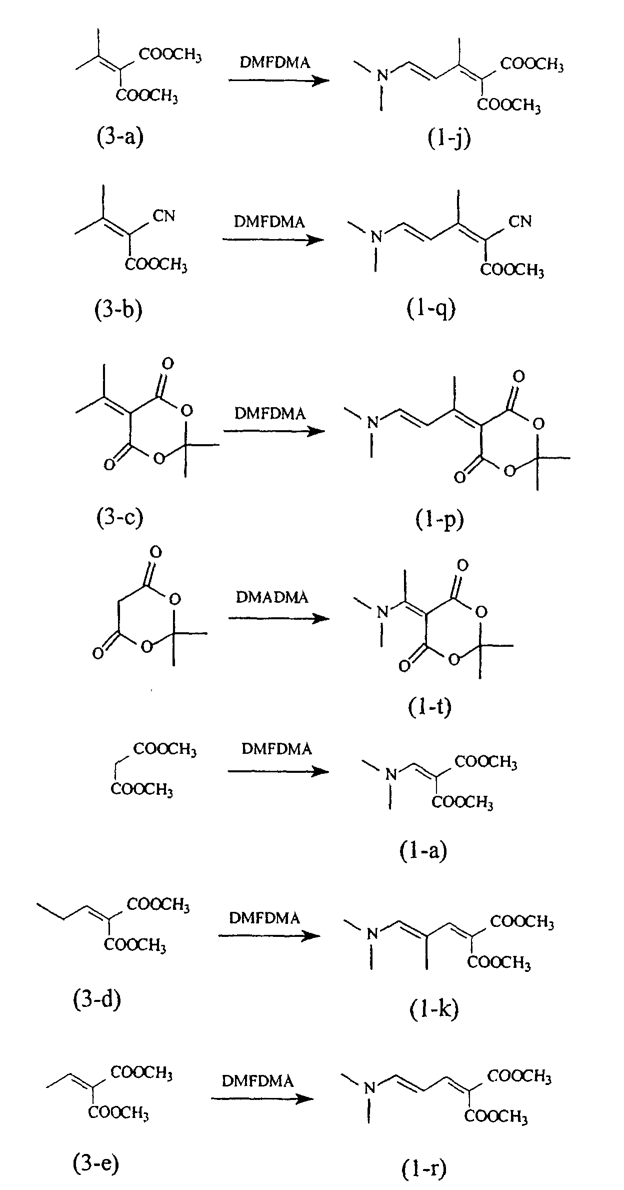

D'autres formes protégées du *N,N*-diméthylformamide
ou des dérivés équivalents, aptes à être mises en oeuvre peuvent être choisis dans le groupe constitué par : 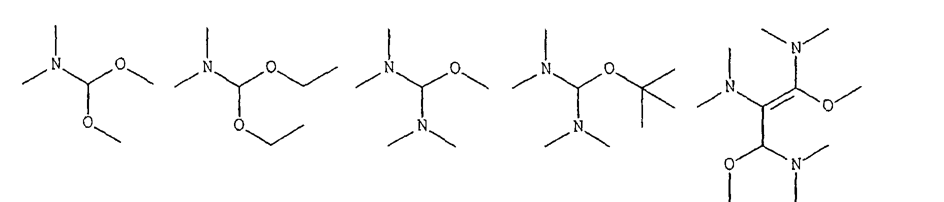 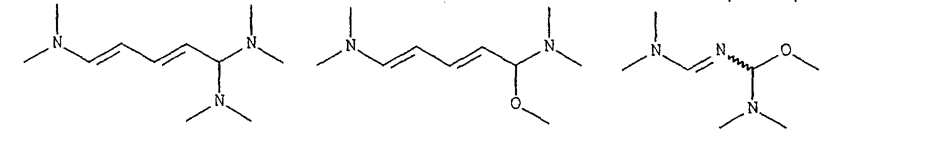 le complexe : *N,N*-diméthylformamide-sulfate de diméthyle; ils peuvent également être préparés par action simultanée ou successive d'orthoformiate d'éthyle ou de méthyle ou d'orthoacétate d'éthyle ou de méthyle et d'une amine secondaire HNR₄R₅.

D'autre part, les composés de formule (1) avec R₁=R₂=H et R₃=CH₃ peuvent être préparés en faisant réagir le diméthylacétal de l'acétylacétaldéhyde avec un composé possédant un méthylène diactivé, en présence d'une amine secondaire, selon le schéma suivant 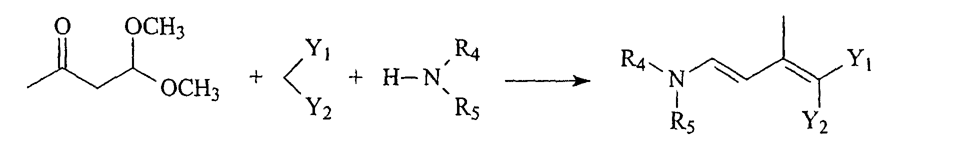 Y₁, Y₂,R₄ et R₅ sont définis tels que précédemment

La présente invention a également pour objet un procédé de synthèse d'un composé de formule (1) comprenant n+1 motifs à partir d'un composé (1) comprenant n motifs, caractérisé en ce qu'on fait réagir dans un solvant, comme par exemple le tétrahydrofurane (THF), le diméthoxyéthane (DME), l'éther éthylique et le tert-butyl-méthyl-éther, un organométallique comme par exemple un halogénure d'alkyl- ou d'arylmagnésium ou un alkyl- ou un aryllithium avec un composé de formule (1) comprenant n motifs, pour obtenir un composé de formule (3) que l'on traite par un composé de formule (2) et on obtient un composé de formule (1) comprenant n+1 motifs.

Dans le cas où n=1, alors le procédé peut être illustré par le schéma qui suit :

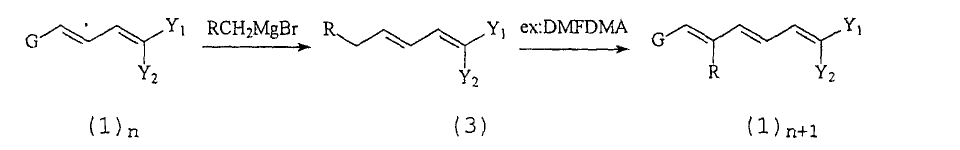

La présente invention a également pour objet un procédé de synthèse d'un composé de formule (1) comprenant n+2 motifs à partir d'un composé de formule (1) comprenant n motifs, caractérisé en ce qu'on fait réagir dans un solvant, comme par exemple le tétrahydrofurane (THF), le diméthoxyéthane (DME), l'éther éthylique et le tert-butyl-méthyl-éther, le composé de formule (1) comprenant n motifs avec l'énolate d'une cétone, de préférence l'acétone ou la butanone, ledit énolate étant généré par une base, comme par exemple l'hydrure de sodium, le diisopropylamidure de lithium, puis on ajoute un organométallique, comme par exemple un halogènure d'alkyl- ou d'arylmagnésium ou un alkyl- ou un aryllithium, et on traite le composé de formule (3) obtenu par un composé de formule (2) et on obtient un composé de formule (1) comprenant n+2 motifs.

Dans le cas où n=1, alors le procédé peut être illustré par le schéma qui suit :

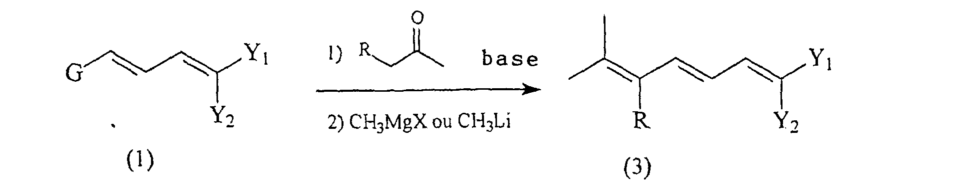

La présente invention a également pour objet, un procédé de préparation d'un aldéhyde de formule (4) 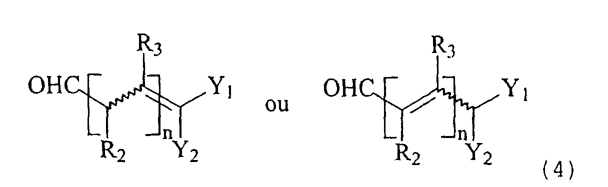 dans lesquelles R₂, R₃, Y₁ et Y₂ sont tels que définis précédemment, caractérisé en ce que l'on fait réagir un composé de formule (1) de préférence dans un milieu acide aqueux (notamment HCl 1 à 2M) ou dans l'acide formique en phase hétérogène.

Dans le cas où n=1, ledit procédé peut être illustré par le schéma suivant:

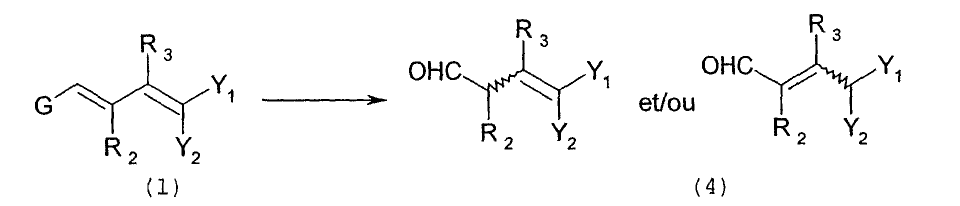

La présente invention a également pour objet, un procédé de synthèse de rétinoïdes caractérisé en ce qu'on utilise comme intermédiaire, un composé de formule (1).

Dans un mode particulier de réalisation dudit procédé, il comprend au moins une étape consistant à faire réagir un composé de formule (1) avec un composé de formule (5) 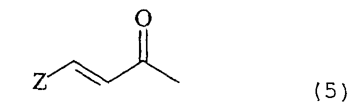 dans laquelle Z est choisi dans le groupe comprenant les radicaux suivants :

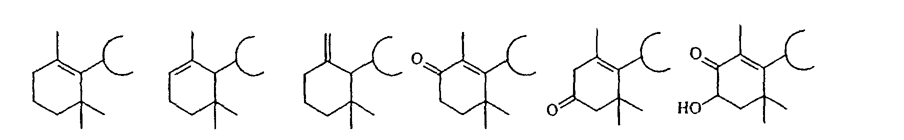

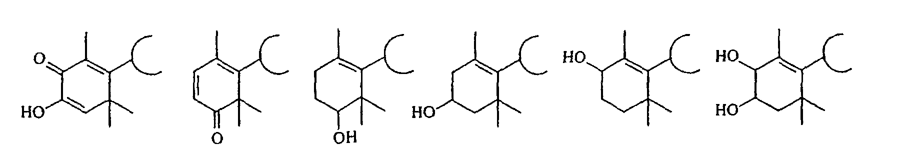

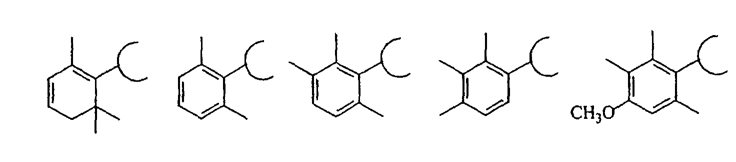

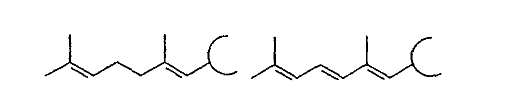

Dans un mode de réalisation particulier de l'invention, la synthèse est réalisée en présence d'une base, choisie de préférence dans le groupe constitué par les amidures, les hydrures et les alcoolates alcalins.

Dans le cas où Z comporte une fonction oxygénée (CO, OH...), une forme protégée de cette fonction (comme définie dans Protective *Groups in Organic Synthesis* (T. W. Greene, 2^{nd} Ed., (1991), Wiley Interscience, Ed.) pourra être utilisée.

Dans un mode très avantageux de réalisation du procédé selon l'invention, la base est le diisopropylamidure de lithium éventuellement associé au *N,N,N',N'*-tétraméthyléthylène-diamine ou le 1,1,1,3,3,3-hexaméthyldisilazane.

Dans un autre mode très avantageux de réalisation du procédé selon l'invention, la réaction est réalisée dans un solvant choisi notamment dans le groupe constitué par le 1,2-diméthoxyéthane (DME), le *tert*-butyl-méthyléther, le tétrahydrofurane (THF), l'éther et leurs mélanges.

Dans un mode particulièrement avantageux de réalisation de l'invention, on fait réagir l'énolate de la β-ionone de formule (5-a) 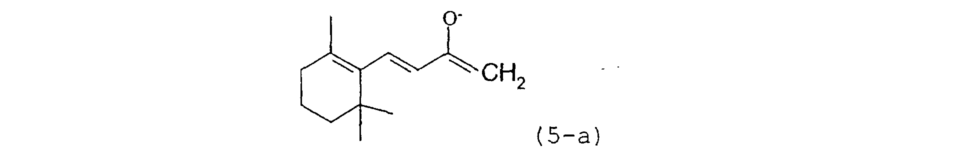 avec le composé de formule (1-j) 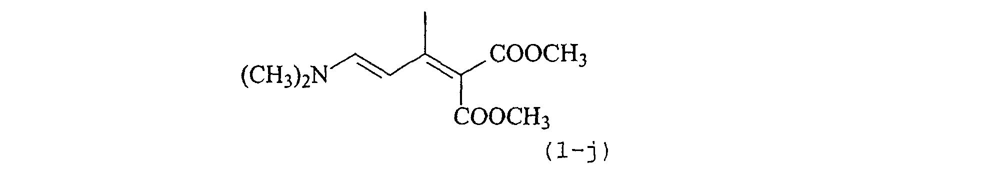 pour obtenir un composé de formule (6-a), sous la forme d'un mélange de 2 isomères.

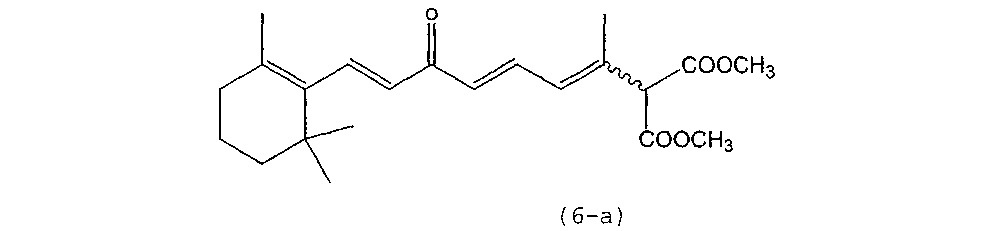

Ce dérivé est traité par un dérivé organométallique (par exemple CH₃MgBr) pour obtenir un composé de formule (7-a), sous la forme d'un mélange de 2 isomères

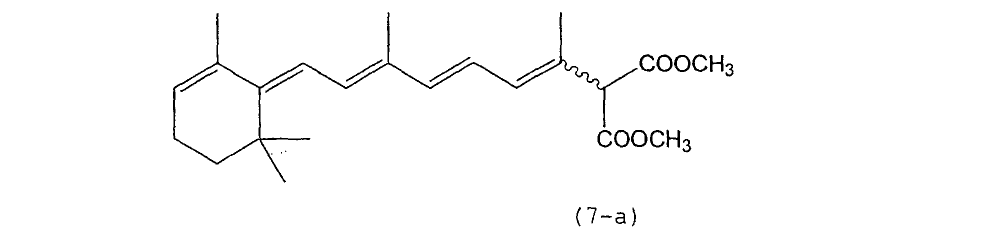

Ce dernier est saponifié, de préférence par une solution hydroalcoolique de potasse ou de soude pour obtenir l'acide rétinoïque *tout trans* de formule (8).

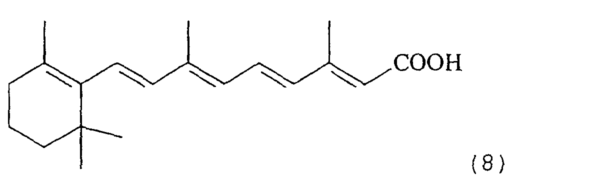

Cet acide est accompagné de son isomère 13-Z, dans des proportions négligeables (<5%). Les traces d'isomère 13-Z sont éliminés lors de la cristallisation de l'acide rétinoïque *tout trans*.

La présente invention a également pour objet un procédé de préparation de la vitamine A:
- *via* le chlorure d'acide de l'acide rétinoïque, selon des méthodes connues de l'homme du métier, notamment selon la méthode décrite par Huisman *et al*., *Recl. Trav. Chim*. Pays-Bas (1956), **75**, 977-1004,
- *via* les esters méthyliques ou éthyliques de l'acide rétinoïque, selon des méthodes connues de l'homme du métier, notamment selon la méthode décrite par Wendler *et al., J. Am. Chem. Soc*. (1949), **71**, 3267 ; Schwarzkoft *et al., Helv. Chim. Acta* (1949), **32**, 443, 451-452 ; Ebeson *et al.,* J. Am. Chem. Soc. (1955), **77**, 4111-18),
ledit procédé étant caractérisé en ce que l'acide rétinoïque est préparé selon le procédé décrit précédemment.

La présente invention a également pour objet l'utilisation des composés de formule (1) comme intermédiaires dans la synthèse des rétinoïdes et des caroténoïdes, notamment dans la synthèse des acides rétinoïques et de la vitamine A.

La présente invention a plus spécifiquement pour objet l'utilisation du composé de formule (1-j) dans la synthèse des rétinoïdes et des caroténoïdes.

Dans un mode de réalisation avantageux selon l'invention, le rétinoïde est choisi dans le groupe constitué par l'acide rétinoïque, la vitamine A (rétinol), le rétinal, le rétinonitrile et l'étrétinate.

Le procédé selon l'invention présente de nombreux avantages techniques et économiques:
- les composés de formule (1) sont facilement préparés, en une ou deux étapes, à partir de matières premières qui sont disponibles dans le commerce, ce qui n'est pas le cas dans les synthèses connues de la littérature ni dans les synthèses industrielles,
- le nouveau synthon énaminodiester de formule (1-j) permet d'obtenir directement, de manière stéréosélective, l'acide rétinoïque *tout trans* par une synthèse multi-étapes '*one pot*', dont le rendement est supérieur à 60% (non optimisé), et
- la préparation se réalise en une seule journée.
   Pour la synthèse de la vitamine A via l'acide rétinoïque *tout trans (via* le chlorure d'acide ou les esters correspondants), celle ci est réalisable en deux journées, ce qui n'est pas le cas des synthèses industrielles utilisées aujourd'hui.
   Les exemples qui suivent illustrent l'invention sans toutefois se limiter à ces modes particuliers de réalisation.
   Les exemples 1a à 1i concernent la préparation des composés de formule (1) comportant (n+1) motifs à partir des composés de formule (3) comportant n motifs.
   Les exemples 2a à 2c concernent la préparation des composés de formule (1) selon le deuxième procédé.
   Les exemples 3a à 3j illustrent le procédé de préparation des composés de formule (3) utiles pour préparer des composés de formule (1) comportant n+1 ou n+2 motifs, à partir des composés de formule (1) comportant n motifs selon les exemples 1a à 1i.
   Les exemples 4a à 4c illustrent l'utilisation des composés de formule (1) pour préparer des aldéhydes correspondants.
   Les exemples 5 à 8 illustrent la synthèse de rétinoïdes à partir des composés de formule (1).

### Exemple 1: Mode opératoire général de préparation des composés de formule 1 selon le premier procédé

A 10 mmoles de diester sont ajoutés 15 mmoles de DMFDMA,
- soit à froid (goutte à goutte), la réaction étant suivie par chromatographie sur couche mince (CCM) et le produit isolé par cristallisation (protocole x),
- soit à la température ambiante, le mélange étant chauffé ensuite directement à une température permettant d'éliminer par distillation le méthanol formé ; la réaction est suivie par chromatographie sur couche mince (CCM). A la fin de la réaction, on élimine le DMFDMA en excès par distillation sous pression réduite (protocole y).

### Exemple 1a: Synthèse du composé (1-a)

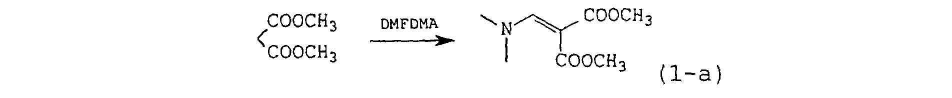

La synthèse est réalisée selon le protocole x. On obtient le produit sous forme de cristaux blancs. Point de fusion = 63°C (éther).

### Exemple 1b: Synthèse du composé (1-j)

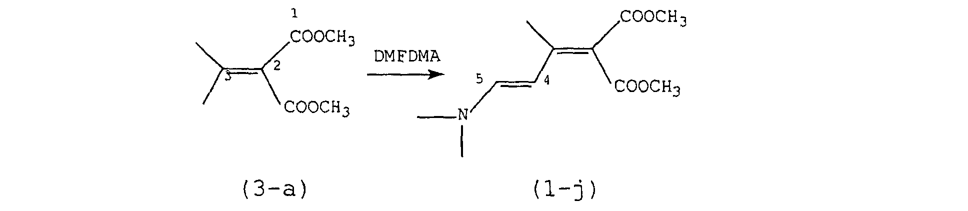

La synthèse est réalisée selon le protocole y. On cristallise l'énaminoester obtenu dans le pentane et on obtient le produit sous forme de cristaux beiges. (Rdt > 85%). (rf: CH₂Cl₂, 0,15). F: 113°C.
En opérant dans 50 mL de diméthylformamide (DMF) et addition de 1 mL d'acide trifluoroacétique, le temps est ramené à 1h d'ébullition du DMF. Rendement > 85%.
IR: 1716; 1686; 1619; 1539; 1433; 1399; 1330; 1191; 1115; 1068; 995; 959.
¹H RMN (CDCl₃): 6, 93 (d, 1H, *J*=13,2, H₄); 6,07 (d, 1H, *J*=13,2, H₅); 3,71 (s, 6H, COOCH₃); 2,91 (s, 6H, N-(CH₃)₂); 2,10 (s, 3H, 3-CH₃).
¹³C RMN (CDCl₃) (CO): 167,3 (CH); 148,8; 97,4 (CH₃): 52,0; 51,9; 41,0; 16,7.

### Exemple 1c: Synthèse du composé (1-q) à partir de (3, R=R₁=CH₃, ester méthylique)

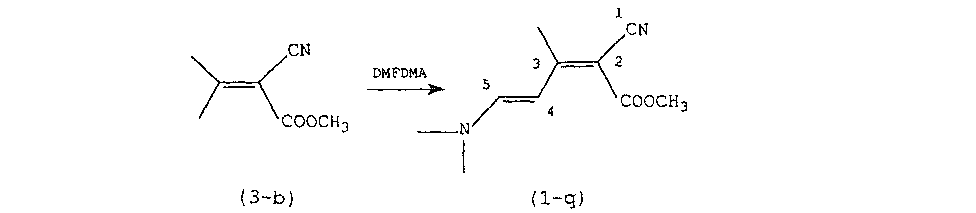

Ce produit est synthétisé selon une méthode dérivée de celle décrite par Köechritz (déjà citée). On mélange 15,3 g (0,01 mole) de 2-cyano-3-méthylcrotonate d'éthyle obtenu selon la méthode décrite par Wideqvist (S. *Acta Chem. Scand*. (1949), **3**, 303), 600 mg d'acide acétique et 11,9 g (0,01 mole) de DMFDMA. On chauffe 2h à 70°C pour éliminer le méthanol puis à 100°C. La réaction est suivie par chromatographie sur couche mince (CCM) (CH₂Cl₂-MeOH 98: 2, *rf* : 0,7). On cristallise l'énaminoester dans l'éther et on l'obtient sous forme de cristaux jaunes. (Rdt > 60%). F: 85°C.
IR: 3145; 2180; 1696; 1615; 1526; 1403; 1273; 1238; 1185; 1137; 842.
¹H RMN (CDCl₃): 7,29 (d, 1H, *J*=13, H₄); 6,1 (d, 1H, *J*=13, H₅); 3,74 (s, 3H, OCH₃); 3,18 et 2,98 (2s aplatis, 6H, (t, 3H,N-CH₃); 2,30 (s, 3H, 3-CH₃).

### Exemple 1d: Synthèse du composé (1-c)

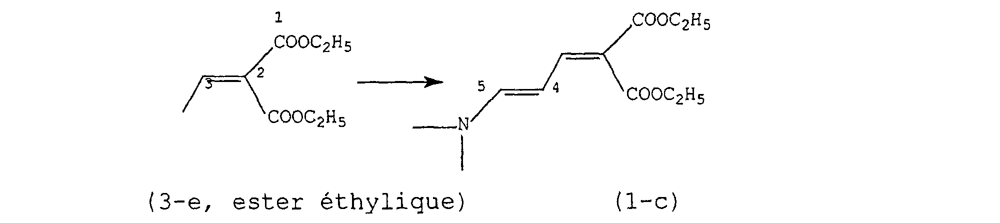

La synthèse est réalisée selon le protocole y. On distille sous pression réduite le diméthylformamide (diméthylacétal) en excès et on cristallise l'énaminoester obtenu dans l'éther. On obtient le produit sous forme de cristaux jaune orangé. (Rdt > 75%). (rf : CH₂Cl₂, 0,15). F: 44°C.
IR: 1684; 1654; 1618; 1555; 1395; 1378; 1218; 1174; 1117; 1071; 1030; 1014.
¹H RMN (CDCl₃): 7,75 (d, 1H, *J*=12,5, H₅); 6,94 (d, 1H, *J*=12,5, H₃); 6,11 (t, 1H, *J*=12,5, H₄); 4,28 et 4,20 (2q, 4H, CH₂); 3,01 (se, 6H, N-(CH₃)_{₂}); 1,33 (2q, 6H, CH₃).
¹³C RMN (CDCl₃) (CO): 167,1 et 166,9 (CH); 157,0; 154,0; 97,5 (CH₂): 60,4; 60,2 (CH₃) 14,8; 14,6.

### Exemple 1e: Synthèse du composé (1-k)

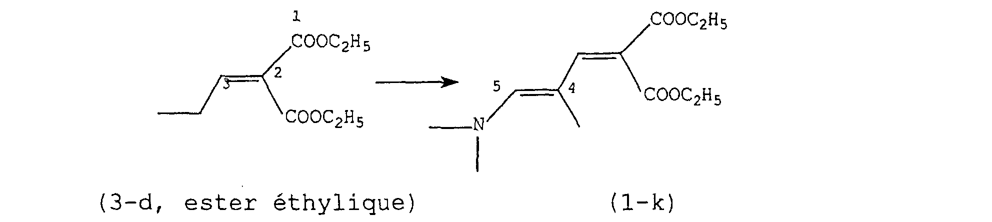

La synthèse est réalisée selon le protocole y. On distille sous pression réduite le diméthylformamide (diméthylacétal) en excès et on cristallise l'énaminoester obtenu dans l'éther. On obtient le produit sous forme de cristaux jaune orangé.
(Rdt > 75%). (rf: CH₂Cl₂, 0,15). F: 44°C.
IR: 1724; 1678; 1619; 1536; 1426; 1390; 1371; 1233; 1169; 1126; 1082; 1023; 918; 881; 761; 726.
¹H RMN (CDCl₃): 7,31 (s, 1H, H₅); 6,79 (s, 1H, H₃); 3,82 et 3,75 (2s, 6H, N-(CH)₃); 3,08 (s, 6H, OCH₃); 1,83 (s, 3H, CH₃).
¹³C RMN (CDCl₃): CH₃: 51,8; 51, 5; 43, 3.

### Exemple 1f: Synthèse du composé (1-p)

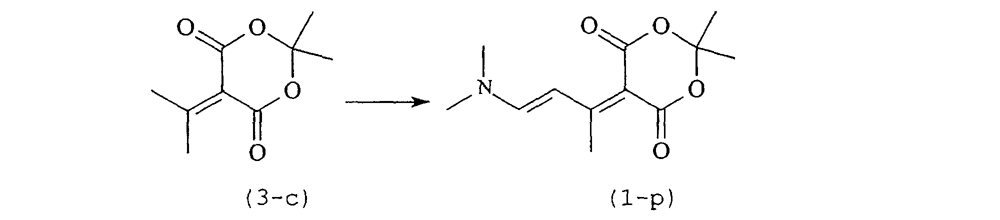

La synthèse est réalisée selon le protocole y. On distille sous pression réduite le diméthylformamide (diméthylacétal) en excès et on cristallise l'énaminoester obtenu dans l'acétate d'éthyle. On obtient le produit sous forme de cristaux orangés.
(Rdt > 80%). (rf: CH₂Cl₂, 0,15). F: 152°C.
IR: 3374; 3415; 1700; 1671; 1596; 1518; 1394; 1318; 1252; 1218; 1199; 1110; 959.
¹H RMN (CDCl₃) : 7,65 et 7,40 (2d, 2H, *J*=12,8, H_{5,4}); 3,27 et 3,08 (2s, 6H, N-(CH₃)₂; 2,54 (s, 3H, 3-CH₃) 1,66 (s, 6H, CH₃).
¹³C RMN (CDCl₃): CO: 169,5; CH: 103, 6 ; CH₃: 46,6; 36,6; 27,0; 19,2.

### Exemple 1g: Synthèse du composé (1-s)

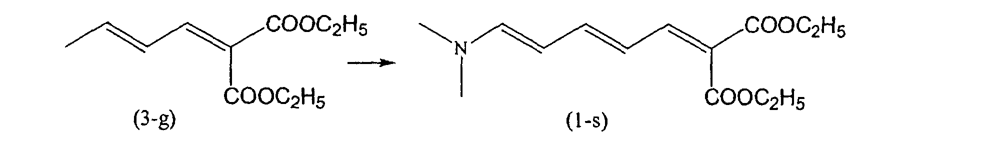

La synthèse est réalisée selon le protocole y. On distille sous pression réduite le diméthylformamide (diméthylacétal). On obtient le produit sous forme d'une huile jaune orangé. Rdt > 75%.
IR: 1725; 1633; 1541; 1375; 1213; 1151; 1100; 1062; 860.
¹H RMN (CDCl₃): 7,53 (d, 1H, *J*=12,3, H₃); 6,85-6,50 (m, 3H, H₄+H₅+H₇); 5,23 (m, 1H, H₆); 4,25 (2q, 4H, CH₂); 2,92 (s, 6H, N-(CH)₃); 1,33 (2t, 6H, CH₃).
¹³C RMN (CDCl₃)(CO) 166,6 et 166,3 (CH) 151,1 ; 151,0 ; 116,4 ;110,0 ;99,7 (CH₃): 14,7.

### Exemple 1h : Préparation du composé (1-t)

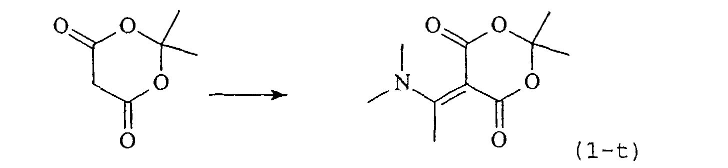

La synthèse est réalisée selon le protocole y avec le diméthylacétamide (diméthylacétal). On distille sous pression réduite le diméthylacétamide (diméthylacétal). On obtient le produit sous forme d'un produit rouge cristallisé. Rdt > 50%.
¹H RMN (CDCl₃): 3,48 (s, 3H, CH₃); 3,23 (s, 6H, N-(CH)₃); 1,71 (s, 6H, C(CH₃)₂.

### Exemple 1i: Synthèse du composé (1-u)

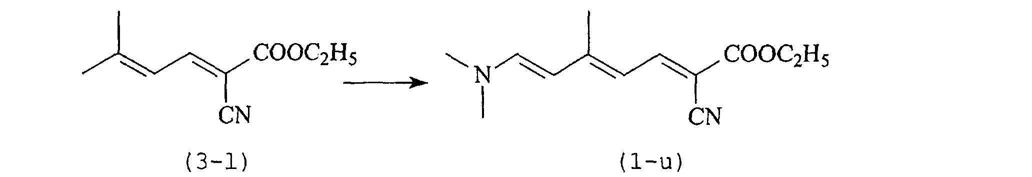

La synthèse est réalisée selon le protocole y. Rdt>40%.
IR: 2925 ; 2200 ; 1707; 1691; 1627; 1516; 1432; 1235; 1182; 1109; 1089 ; 1019.
¹H RMN (CDCl₃): 8,18 (d, 1H, *J*=13,2); 7,04 (d, 1H, *J*=13); 6,38 (d, 1H, *J*=13,2); 5,38 (d, 1H, *J*=13); 3,83 (s, 3H); 3,03 (s, 6H) ; 1,59 (s, 3H).
¹³C RMN (CDCl₃)(CO) 165,7 ; (CN) 114,7; (CH) 151,0 ; 481,5 ; 117,7 ;114,7 (CH₃): 52,0 ; 13,9.

### Exemple 2: Mode opératoire général de préparation des composés de formule (1) selon le deuxième procédé

### Exemple 2a : Préparation du composé (1-v)

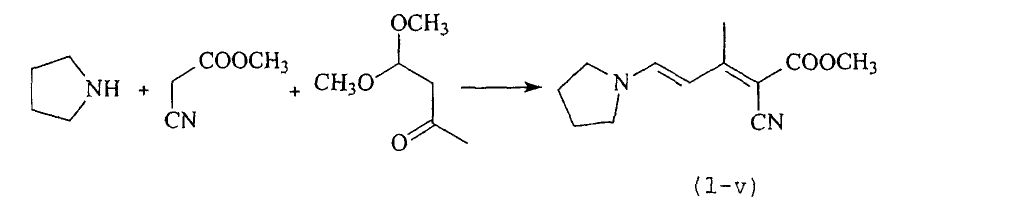

Sous argon, 11 mmoles de cyanoacétate de méthyle, 10 mmoles de diméthylacétal de l'acétylacétaldéhyde et 12 mmoles de pyrrolidine dans 100 mL de toluène sont agités 1 h à la température ordinaire puis au reflux 6 h avec un appareil de type Dean-Stark. Le produit cristallise en refroidissant et est recristallisé dans un mélange éther-méthanol. Cristaux jaunes F 162-64°C. Rdt >65%.
IR: 3460 ; 2965 ; 2189 ; 1679; 1570; 1454; 1230.
¹H RMN (CDCl₃) : 8,07 (d, 1H, *J*=13,5); 5,56 (d, 1H, *J*=13,5 ); 3,77 (s, 3H); 3,59 (m, 2H); 3,46 (m, 2H); 2,25 (s, 3H); 2,05 (m, 4H).
¹³C RMN (CDCl₃)(CO) 166,6 (CH) 152, 9 ; 97,9 ; CN 82, 7; (CH₂) 49, 7; 49,1; 25,1 ; 24,7 (CH₃): 51, 6; 16, 7.

### Exemple 2b : Préparation du composé (1-w)

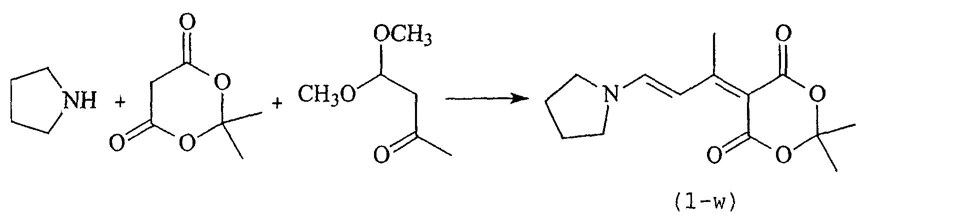

Sous argon, 11 mmoles d'acide de Meldrum, 10 mmoles de diméthylacétal de l'acétylacétaldéhyde et 12 mmoles de pyrrolidine dans 100 mL de toluène sont agités 1 h à la température ordinaire puis au reflux 6 h avec un appareil de type Dean-Stark. Le produit cristallise en refroidissant et est recristallisé dans un mélange éther-méthanol. Cristaux rouges F 218-22°C (dec). Rdt >35%.
IR: 3440 ; 2978 ; 1668; 1558; 1359; 1188.
¹H RMN (CDCl₃): 8,24 (d, 1H, *J*=14); 7, 05 (d, 1H, *J*=14 ); 3,68 (m, 4H); 2,36 (s, 3H); 2,08 (m, 4H); 1,70 (s, 6H).
¹³C RMN (CDCl₃) (CO) 166, 9; 166,1 (CH) 152,0 ; 103,8 ; (CH₂) 50,5; 50,1; 24, 9 ; 24,5 (CH₃) : 25,8; 16,5.

### Exemple 2c: Synthèse du composé (1-q) selon le procédé précedent avec la diméthylamine au lieu de la pyrrolidine

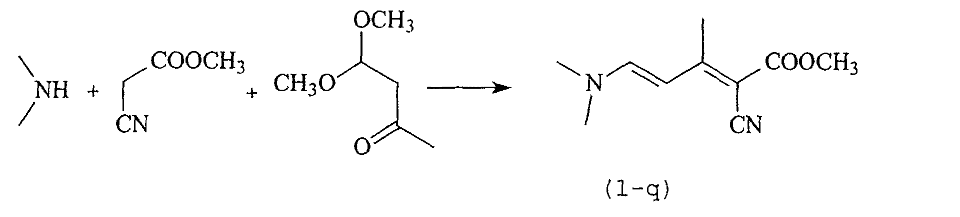

On cristallise l'énaminoester dans l'éther et on l'obtient sous forme de cristaux jaunes. (Rdt > 50%).

### Exemple 3a: Premier procédé (n motifs à n+1 motifs)

Sous argon, 10 mmoles d'un halogénure d'alkylmagnésium, par exemple de chlorure ou de bromure de méthylmagnésium (3 M dans le tétrahydrofurane (THF)) sont ajoutés à une température voisine de -10°C, à 10 mmoles d'énamine (1) comprenant n motifs, par exemple le composé (1-j) dans 20 mL de THF. On laisse 1h30 à la température ambiante. On refroidit vers -0°C et on ajoute 1 mL d'éthanol. On acidifie légèrement par HCl 1M et on extrait les impuretés à l'éther. On passe en milieu basique par addition d'ammoniaque, vers 0°C. On extrait au dichlorométhane, lave à l'eau et sèche sur sulfate de sodium. Le produit brut est dissous dans 15 mL de toluène et chauffé 30 mn à ébullition. Après évaporation sous pression réduite, le produit est isolé. Ce produit, traité par le DMFDMA permet de préparer le composé de formule (1) comprenant n+1 motifs.

### Exemple 3b: Second procédé (n motifs à n+2 motifs)

Sous argon, 10 mmoles de butyllithium sont ajoutés à une température voisine de -25°C, à 10 mmoles de diisopropylamine dans 20 mL de THF. On ajoute lentement 10 mmoles de cétone, notamment l'acétone dans 15 mL de THF. On laisse l'agitation pendant 10 min. et on ajoute, vers -30°C, 10 mmoles d'énamine (1) comprenant n motifs ou d'éther d'énol (1) comprenant n motifs, dans 20 mL de THF. On laisse revenir à température ambiante en 10 minutes et on porte 2h30 à reflux (jusqu'à ce que le dégagement de diméthylamine cesse). On refroidit à une température comprise entre -10 et 0°C et on ajoute 20 mmoles de chlorure de méthylmagnésium (3M dans le THF). On laisse revenir à la température ambiante. Après 1h30 d'agitation, on ajoute, à 0°C, 1 mL d'éthanol, puis on acidifie par HCl 1M. On extrait au dichlorométhane, lave à l'HCl 1M puis à l'eau. Après séchage sur MgSO₄, on isole le produit après distillation des solvants sous pression réduite. Ce produit, traité par le DMFDMA permet de préparer le composé de formule (1) comprenant n+2 motifs.

### Exemple 3c:Synthèse du composé (3-f)

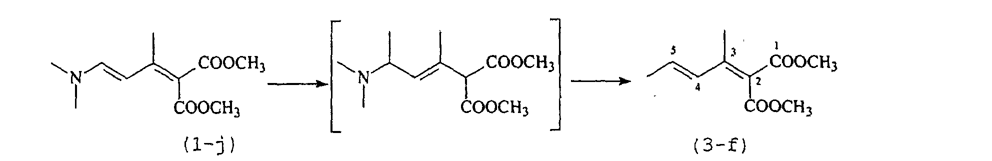

Partant du composé (1-j), on utilise le procédé de l'exemple 3a.
Huile incolore. Rdt 90%.
IR : 2960 ; 1723 ; 1638 ; 1591 ; 1435 ; 1307 ; 1224 ; 1065.
¹HRMN (CDCl₃) : 6,77 (dq, 1H, J=15,5, j'=1,6, H₄ ; 6,32 (dq, 1H, J=15,5, j'=6,8, H₅ ; 3,77 et 3,78 (2s, 6H,COOCH₃) ; 2,15 (s, 3H, 3-CH₃) ; 1,89 (dd, 3H, J=6,8, J'=1,6, 5-CH₃).
¹³CRMN (CDCl₃) : (CO) : 166,2 ; 166,1 (CH) : 136,4 ; 129,6 (CH₃) : 52,4 ; 52,1 ; 19,1 ; 16,0.

### Exemple 3d: Synthèse du composé (3-e) (exemple 5)

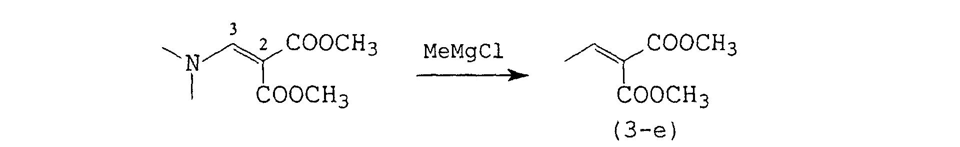

Partant du composé préparé à l'exemple 2, on utilise le procédé de l'exemple 3a.
Chromatographie rapide sur silice *rf* : 0 ,8 (éluant : dichlorométhane). Huile incolore Rdt purifié (80%).
IR (film): 2955 ; 1734 ; 1646 ; 1437 ; 1382 ; 1359 ; 1265 ; 1225 ; 1128 ; 1056.
¹H RMN (CDCl₃) : 7,10 (q, 1H, J=7, 3, H₃) ; 3,80 (2s, 6H, OCH₃) ; 1,93 (d, 3H, , J=7,3 4-CH₃).
¹³C RMN (CDCl₃) (CO): 165,6; 164,2 (CH): 145,6 (CH2): 61,0 (CH₃): 52,3; 15,5.

### Exemple 3e: Synthèse du composé (3-g)

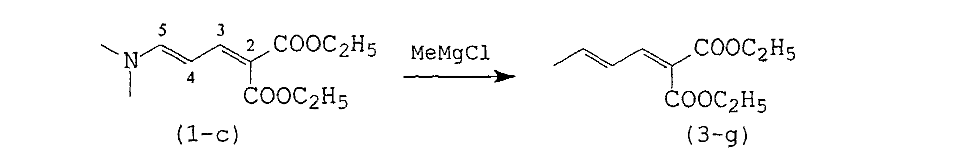

Partant du composé (1-c), on utilise le procédé de l'exemple 3a.
Chromatographie rapide sur silice *rf* : 0 ,8 (éluant : dichlorométhane). Huile incolore Rdt purifié (80%). Mélange d'isomères (1 très majoritaire, >95%).
IR (film): 2984 ; 2939 ; 1725 ; 1641 ; 1604 ; 1447 ; 1373 ; 1300 ; 1242 ; 1213 ; 1170 ; 1095 ; 1024 ; 979.
¹H RMN (CDCl₃) : 7,35 (d, 1H, J=11, 5, H₃) ; 6,51 (dd, 1H, J=11, 5, J=1,7 H₄) ; 6,35 (m, 1H, H₅) ; 4,30 (2q, 2H, J=7,2, CH₂) ; 1,91 (d, 3H, , J=6,8 6-CH₃) ; 1,32 (2t, 3H, J=7,2, CH₃) ;
¹³C RMN (CDCl₃) (CO) : 165,3 ; 164,7 (CH) : 145,0 ; 144,2 ; 127,0 (CH2) : 61,0 (CH₃) : 19,0 ; 14,0.

### Exemple 3f: Synthèse du composé (3-h)

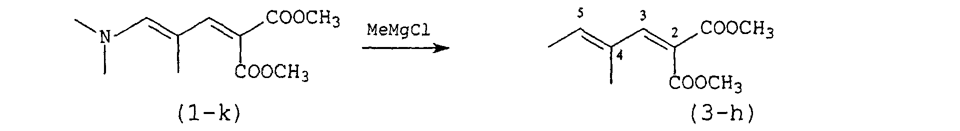

Partant du composé (1-k), on utilise le procédé de l'exemple 3a.
Chromatographie rapide sur silice *rf* : 0 ,8 (éluant : dichlorométhane). Huile incolore Rdt purifié (80%).
IR (film): 2953 ; 1735 ; 1622 ; 1607 ; 1436 ; 1374 ; 1263 ; 1221 ; 1074 ; 1038.
¹H RMN (CDCl₃) : 7,25 (s, 1H, H₃) ; 6,11 (q, 1H, *J*=7,1, H₅) ; 3,79 et 3,74 (2s, 6H, OCH₃) 1,78 (d, 3H , *J*=7,1, 6-CH₃) ; 1, 70 (s, 3H, 4-CH₃).
¹³C RMN (CDCl₃) (CO) : 167,5 ; 165,0 (CH) : 147,1 ; 140,8 (CH₃) : 52,2 ; 14,6 ; 12,1.

### Exemple 3g: Synthèse du composé (3-i)

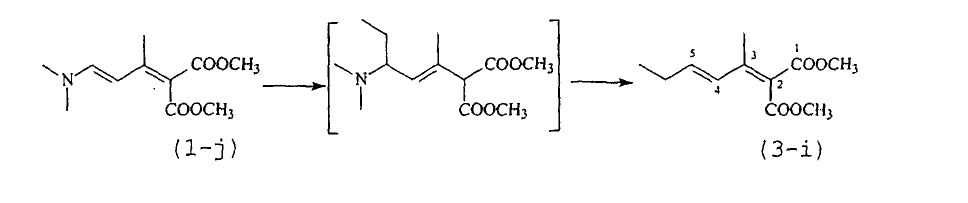

Partant du composé (1-j), on utilise le procédé de l'exemple 3a.
Chromatographie rapide sur silice *rf* : 0 ,8 (éluant : dichlorométhane. Huile incolore. Rdt 90%.
¹HRMN (CDCl₃) : 6,77 (dt, 1H, *J*=15,5, *j'*=1,5, H₄ ; 6, 34 (dt, 1H, *J*=15,5, *j'*=6,7, H₅ ; 3,80 et 3,78 (2s, 6H,COOCH₃) ; 2,22 (2dq, 2H, CH₂, *J*=7,5, *J*=1,5) ; 2,17 (s, 3H, 3-CH₃) ; 1,06 (t, 3H, *J*=7,5,6-CH₃).
¹³CRMN (CDCl₃) : (CO) : 166,3 ; 166,1 (CH) : 142,9 ; 127,3 (CH₂) : 26,4 (CH₃) : 52,1 ; 52,0 ; 16,0 ; 12,9.

### Exemple 3h: Synthèse du composé (3-j)

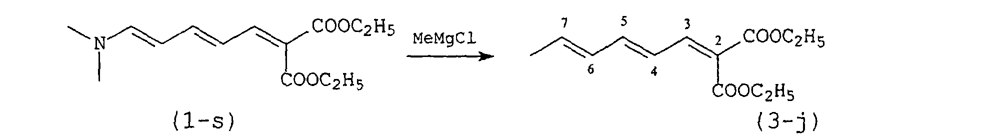

A partir du composé obtenu dans l'exemple 1g, on utilise le procédé de l'exemple 3a.
Chromatographie rapide sur silice *rf*: 0,8 (éluant : dichlorométhane). Huile incolore Rdt purifié (80%).
IR (film): 2953; 1734; 1700; 1622; 1607; 1436; 1373; 1263; 1222; 1075; 1038.
¹H RMN (CDCl₃): 7,37 (d, 1H, *J*=11,6, H₃); 6,64 (m, 1H, H₅); 6,56 (m, 1H, H₄); 6,18 (m, 1H, H₆); 6,05 (m, 1H, H₇); 4,24 (2q, 2H, *J*=7,1, CH₂); 1,84 (d, 3H, *J*=6,8, 6-CH₃); 1,32 (2t, 3H, *J*=7,1, CH₃).
¹³C RMN (CDCl₃) (CO): 165,3; 164,7 (CH): 145,3; 145, 0; 137,6; 131,3; 123,8(CH2): 61,0 (CH₃): 18,6; 14,0.

### Exemple 3i: Synthèse de l'isomère déconjugué du composé (3-k) (ester méthylique)

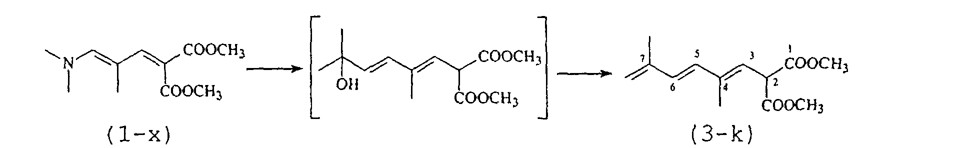

Partant du composé (1-x), on utilise le procédé de l'exemple 3b.
Huile incolore. Rdt 60%.
IR : 2954 ; 1740 ; 1738 ; 1640 ; 1610 ; 1435 ; 1311 ; 1259 ; 1224 ; 1195 ; 1149 ; 1021 ; 962.
¹HRMN (CDCl₃) : 6,38 (d, 1H, *J*=16, H₅); 6,29 (d, 1H, *J*=16, H₆ ; 5,81 (d, 1H, *J*=9,6, H₃) ; 5,03 (2s, 2H, CH₂) ; 4,44 (d, 1H, *J*=9,6,H₂) ; 3,75 (s, 6H,COOCH₃) ; 1,88 (s, 3H, 7-CH₃) ; 1,86 (s, 3H, 4-CH₃).
¹³CRMN (CDCl₃) : (CO) : 168,4 (CH) : 132, ; 131,8 ; 122,2 ; 51,4 (CH₂) : 117,3 (CH₃) : 52,7 ; 18,4 ; 18,0.

### Exemple 3j: Synthèse du composé (3-1)

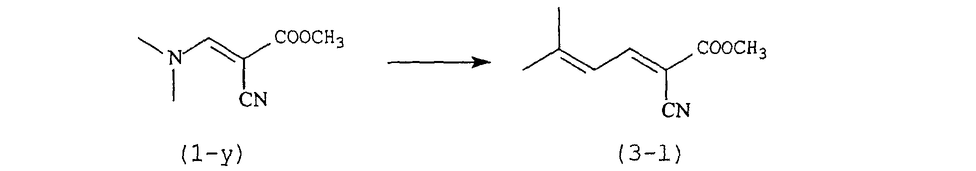

Le composé (1-y) est obtenu selon la technique employée par H. Merwein *Justus Liebigs Ann. Chem*. 1961, 641, 1-39. A partir de celui-ci, le composé (3-1) est obtenu selon le procédé décrit dans l'exemple 3b.
Cristaux beiges Rdt >40%). F 64-66°C
IR (film) : 2958 ; 2220; 1748 ; 1724 ; 1620 ; 1575 ; 1437 ; 1264 ; 1163 ; 1093 ; 762.
¹H RMN (CDCl₃) : 8,16 (d, 1H, *J*=12,3) ; 6, 52 (d, 1H, *J*=12,3) ; 3,89 (s, 3H) ; 2,06 (s, 3H) ; 2,02 (s, 3H). ¹³C RMN (CDCl₃) (CO) : 163,3 ; 114,6 (CN) ; (CH) : 151,5 ; 122,7 (CH₃) : 52,8,6 ; 27,4 ; 20,0.

### Exemple 4a: Procédé de transformation d'un composé de formule (1-x) en aldéhyde correspondant (4-a).

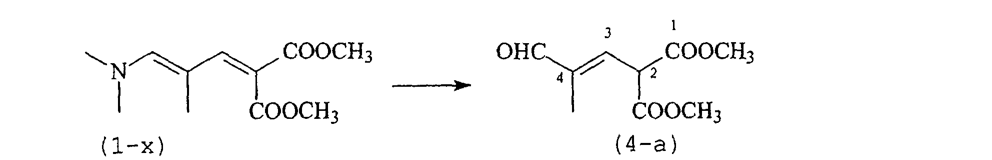

0,01 mole d'énaminoester (1-x) dans 40 mL de dichlorométhane et 40 mL d'HCl 1M sont agités 6h à la température ambiante. La phase organique est lavée à l'eau et séchée sur MgSO₄. Après distillation du dichlorométhane sous pression réduite, on obtient une huile incolore. Rdt 95%.
IR : 3006 ; 2958 ; 2847 ; 1739 ; 1695 ; 1649 ; 1599 ; 1437 ; 1317 ; 1264 ; 1219 ; 1154 ; 1017.
¹HRMN (CDCl₃) : 9,49 s, 1H, CHO) ; 6,72 (dq, 1H, *J*=9,4, *J*'=1,4, H₃) ; 4,54 (d, 1H, *J*=9,4, H₂ ; 3,78 (s, 6H, CH₃) ; 1,79 (d, 3H, 4-CH₃).
¹³CRMN (CDCl₃) : (CO) : 193,9 (CH) : 141,6 ; 53,1 ; (CH₃) : 51,5 ; 9, 5.

### Exemple 4b: Procédé de transformation d'un composé de formule (1-j) en aldéhydes correspondants (4-b) et (4-c)

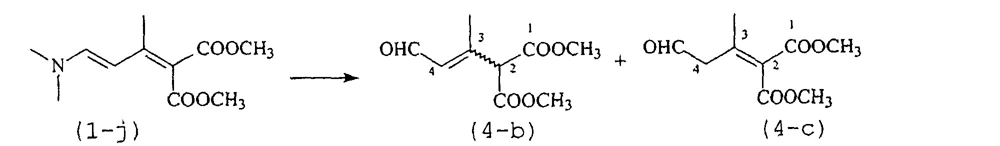

a) 0,01 mole d'énaminoester dans 40 mL de dichlorométhane et 40 mL d'HCl 2M sont agités 15h à la température ambiante. La phase organique est lavée à l'eau et séchée sur MgSO₄. Après distillation du dichlorométhane sous pression réduite, on obtient une huile incolore, constituée de 3 isomères. Rdt 95%.
b) 0,01 mole d'énaminoester en suspension dans 40 mL de cyclohexane et 10 mL d'acide formique sont agités 2h à la température ambiante. On ajoute 100 mL d'eau glacée et extrait la phase aqueuse à l'éther. On lave les phases organiques à l'eau et on sèche sur MgSO₄. Après distillation des solvants sous pression réduite, on obtient une huile incolore, constituée de 3 isomères Rdt=(50%).

### Exemple 4c: Procédé de transformation d'un composé de formule (1-u) en aldéhyde correspondant (4-d)

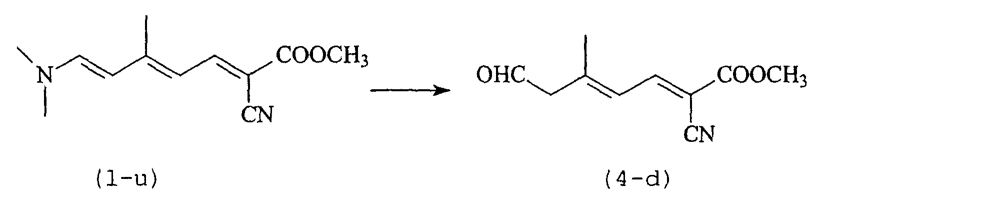

0,01 mole d'énaminoester dans 40 mL de dichlorométhane et 40 mL d'HCl 2M sont agités 18 h à la température ambiante. La phase organique est lavée à l'eau et séchée sur MgSO₄. Après distillation du dichlorométhane sous pression réduite, on obtient une huile incolore. Rdt >25%.
IR (film): 2958 ; 2225; 1734 ; 1691 ; 1580 ; 1436 ; 1264 ; 1087 ; 1015 ; 801 ; 703.
¹H RMN (CDCl₃) : 9,73 (s, 1H) ; 8, 32 (d, 1H, *J*=12) ; 7, 33 (d, 1H, *J*=12) ; 3,97 (s, 3H) ; 3,35 (s, 2H) ; 2,09 (s, 3H).
¹³C RMN (CDCl₃) (CO) : 193,4 ; (CH) : 147,8 ; 137,8 (CH₂): 53,5 (CH₃) : 53,6,6 ; 10,9.

### Exemple 5: Procédé général de synthèse des rétinoïdes.

Sous argon, on ajoute à une température voisine de -25°C, 10 mmoles de butyllithium à 10 mmoles de diisopropylamine dans 20 mL de diméthoxyéthane (DME). On ajoute lentement 10 mmoles de β-ionone dans 20 mL de DME, à une température comprise entre -30 et -40°C. On laisse l'agitation pendant 20 minutes et on ajoute 10 mmoles de dérivé (1), dans laquelle G représente un groupe -NR₄R₅ ou OP, dans 50 mL de DME. On laisse revenir à la température ambiante en 10 minutes et on porte 1h à reflux (jusqu'à ce que le dégagement de diméthylamine cesse). On refroidit à une température comprise entre -10 et 0°C et on ajoute 20 mmoles, halogénure d'alkyl- ou d'arylmagnésium (3M dans le THF). On laisse revenir à la température ambiante en 30 minutes et on ajoute, à 0°C, 15 mL d'éthanol puis 5 équivalents de potasse dans 50 mL d'eau. On agite 30 minutes à la température ambiante et 45 minutes à 40°C. On distille les solvants organiques sous pression réduite. On acidifie par une solution d'acide chlorhydrique 2N glacée. On extrait à l'acétate d'éthyle, on lave avec de l'acide chlorhydrique 1M, puis on lave à l'eau et on sèche sur sulfate de sodium.

### Exemple 6: Synthèse 'one pot' de l'acide rétinoïque (acide 3,7-diméthyl-9-(2,6,6-triméthyl-cyclohex-1-enyl)-nona-2,4,6,8-tétraénoïque).

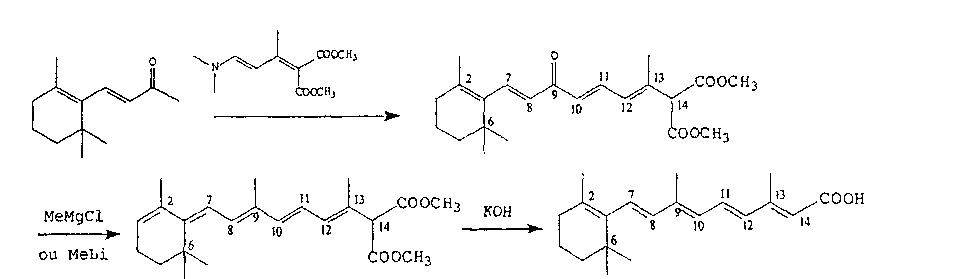

On fait réagir la β-ionone avec le composé (1-j) préparé à l'exemple 1b selon le procédé général décrit dans l'exemple 5.
Le rendement après purification est égal à 61%.
Les caractéristiques physico-chimiques sont identiques à celles d'un échantillon de référence (SIGMA).

### Exemple 7: Préparation de l'analogue 9,12-diméthyle de l'acide rétinoïque tout trans (acide 4,7-diméthyl-9-(2,6,6-triméthyl-cyclohex-1-enyl)-nona-2,4,6,8-tétraénoïque).

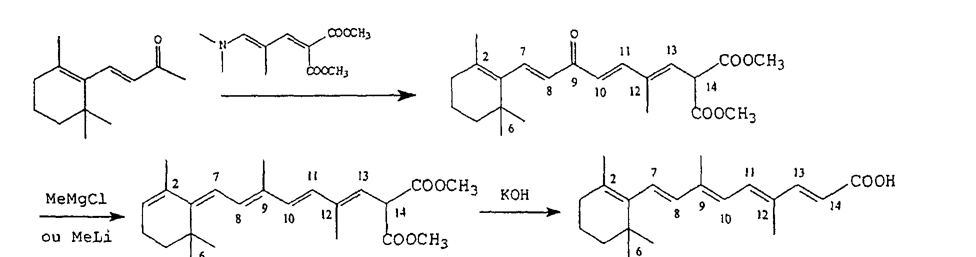

On fait réagir la β-ionone avec le composé (1-x) selon le procédé général décrit dans l'exemple 5.
CCM : rf: 0,3 (CH₂Cl₂/MeOH 95/5).
Le rendement en produit brut est égal à 75%.
Le produit brut est extrait par une solution saturée de bicarbonate. Après acidification et extraction à l'éther, le produit obtenu est purifié par chromatographie sur silice et obtenu sous forme d'un liquide jaune,
Le rendement en produit purifié est égal à 25%.
IR: 2948; 2850; 1715; 1601; 1500; 1460; 1028; 756; 697.
¹H RMN (CDCl₃): 7,53 (d, 1H, *J*=15,4, H₁₃); 6,83 (d, 1H, *J*=12,1, H₁₃); 6,38 (m, 2H, H₇₊₁₀); 6,23 (d, 1H, *J*=15,1, H₈ ); 5, 87 (d, 1H, *J*=15,4, H₁₄); 2, 03 (s, 3H, 12-CH₃); 1, 95 (s, 3H, 9-CH₃); 1,94 (m, 2H, 3-CH₂); 1,80 (s, 2H, 4-CH₂); 1,75 (s, 3H, 3-CH₃); 1,67 (m, 2H, 5-CH₂); 1,05 (s, 3H, 6-CH₃).
¹³C RMN (CDCl₃) (CO): 185,6 (CH): 151,6; 137,6; 137,4; 136,1; 130,3; 125,4 (CH₂): 39,5; 34,2; 19,1 (CH₃): 30,9; 28,9 ; 12,7.

### Exemple 8: Préparation de l'analogue 13-desméthyle de l'acide rétinoïque (acide 7-méthyl-9-(2,6,6-triméthylcyclohex-1-enyl)-nona-2,4,6,8-tétraénoïque).

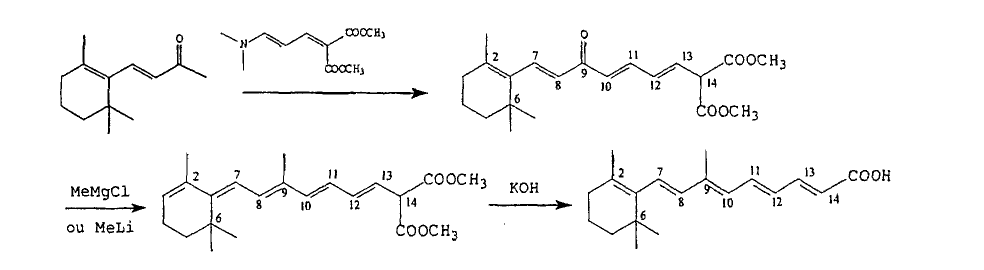

On utilise le procédé général décrit à l'exemple 5. CCM : *rf*: 0,35 (CH₂Cl₂/MeOH 95/5).Rdt après purification 7g (52%).
On obtient le produit sous forme d'un mélange d'isomères dans lequel l'isomère *tout trans* est très majoritaire.

## Revendications

1. Composés de formule (1) 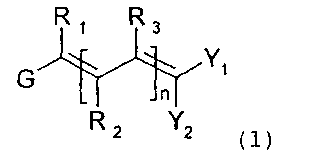 dans laquelle
- G représente un groupe -NR₄R₅ où R₄ et R₅, identiques ou différents, sont chacun un groupe alkyle de 1 à 5 atomes de carbone, linéaire ou ramifié, ou un groupe cycloalkyle de 3 à 7 atomes de carbone, ou R₄ et R₅ forment un cycle avec l'atome d'azote qui les porte,
- R₁ représente, soit un atome d'hydrogène, soit un groupe alkyle de 1 à 5 atomes de carbone, linéaire ou ramifié,
- n est un nombre entier compris entre 1 et 2,
- R₂ et R₃ représentent un substituant semblable ou différent dans chacun des motifs insaturés, lesdits motifs insaturés successifs pouvant être par ailleurs identiques ou différents, R₂ et R₃ étant choisis dans le groupe comprenant l'hydrogène et les groupes alkyles de 1 à 5 atomes de carbone, linéaires ou ramifiés, et à condition qu'au moins un des substituants R₁, R₂ et R₃ soit différent de H, et
Y₁ et Y₂ représentent chacun un groupe -COOR₇ où R₇ est un groupe alkyle de 1 à 5 atomes de carbone.

2. Composés selon la revendication 1, **caractérisés en ce que**:
- G représente le groupe -N (CH₃)₂, -N(C₂H₅)₂ ou N-pyrrolidine,
- n est un nombre entier égal à 1 ou 2,
- R₁, R₂ et R₃ représentent, indépendamment l'un de l'autre, un atome d'hydrogène ou un groupe méthyle, les motifs insaturés successifs pouvant être par ailleurs identiques ou différents, à condition qu'au moins un des substituants R₁, R₂ et R₃ soit différent de H, et
- Y₁ et Y₂ représentent chacun un groupe -COOCH₃ ou un groupe -COOC₂H₅.

3. Composés selon l'une quelconque des revendications 1 et 2, **caractérisés en ce qu'**ils sont choisis dans le groupe constitué par : 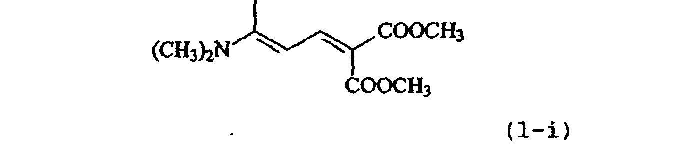 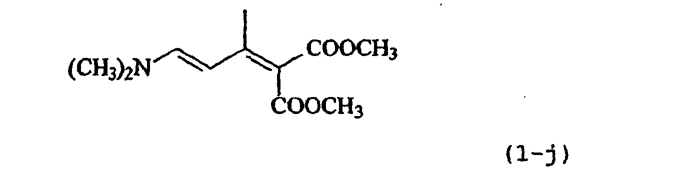 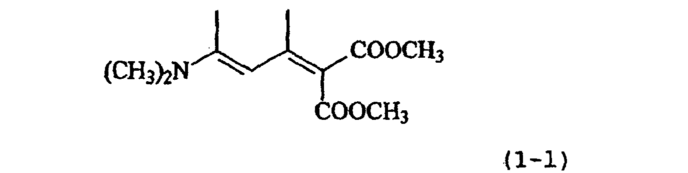 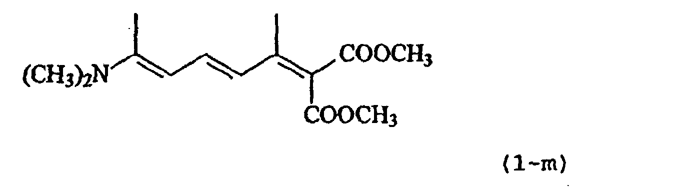 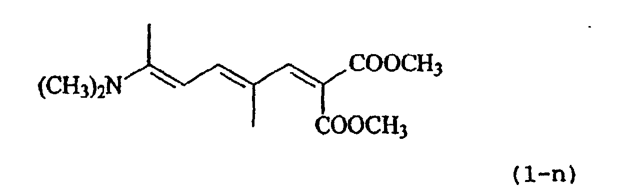 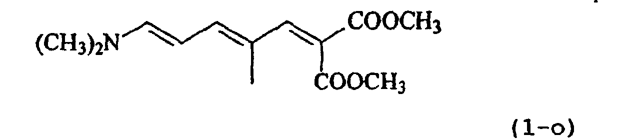 et les analogues esters éthyliques des composés (1-i) à (1-j) et (1-1) à (1-o).

4. Préparation du composé (1-j) selon la revendication 3, **caractérisée en ce qu'**on fait réagir l'isopropylidène malonate de méthyle de formule (3-a) 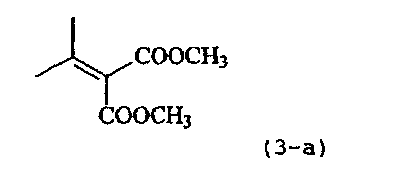 avec une forme protégée du *N,N*-diméthylformamide, notamment le DMFDMA de formule (2-a)

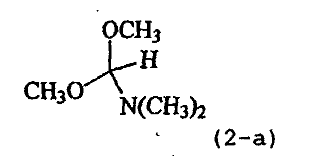

5. Procédé de synthèse des composés 1-i, 1-l, 1-m et 1-n selon la revendication 3, **caractérisé en ce que** l'on fait réagir le composé de formule (3) 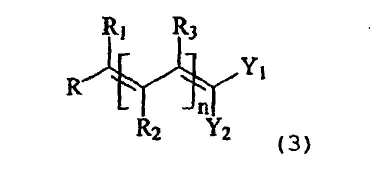 dans laquelle R₁, R₂ et R₃, Y₁ et Y₂ sont tels que définis précédemment et R représente un groupe alkyle (de 1 ou 2 atomes de carbone) correspondant, avec le composé de formule (2-b)

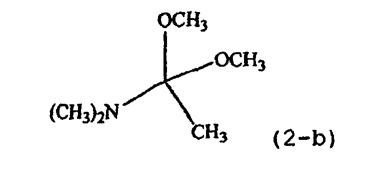

6. Procédé de synthèse d'un composé de formule (1) selon l'une quelconque des revendications 1 à 3 comprenant n+1 motifs, à partir d'un composé de formule (1) comprenant n motifs, **caractérisé en ce que** l'on fait réagir dans un solvant, un organométallique avec un composé de formule (1) comprenant n motifs, pour obtenir un composé de formule (3) que l'on traite par un dérivé de *N,N*-diméthylformamide ou d'acétamide, de préférence par un composé de formule (2-a) ou (2-b).

7. Procédé de synthèse d'un composé de formule (1) comprenant n+2 motifs à partir d'un composé (1) comprenant n motifs, **caractérisé en ce qu'**on fait réagir dans un solvant, un composé de formule (1) comprenant n motifs avec l'énolate d'une cétone (de préférence de l'acétone ou de la butanone), ledit énolate étant généré par une base, puis on ajoute un organométallique, de préférence un halogènure d'alkyl- ou d'arylmagnésium ou un alkyl- ou un aryllithium et on traite le composé de formule (3) obtenu par un dérivé de *N,N*-diméthylformamide ou d'acétamide, de préférence par un composé de formule (2-a) ou (2-b).

8. Procédé de synthèse de rétinoïdes, **caractérisé en ce qu'**on utilise comme intermédiaire, un composé de formule (1) selon l'une quelconque des revendications 1 à 3.

9. Procédé selon la revendication 8, **caractérisé en ce qu'**il comprend au moins une étape consistant à faire réagir un composé de formule (1) avec un composé de formule (5) 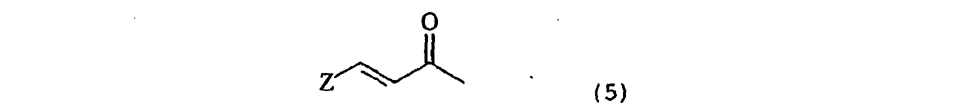 dans laquelle Z est choisi dans le groupe comprenant les radicaux suivants :

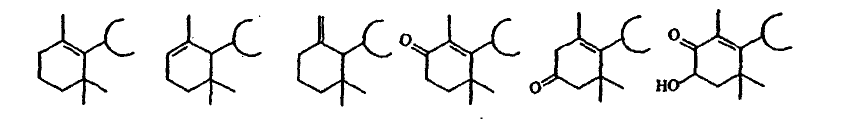

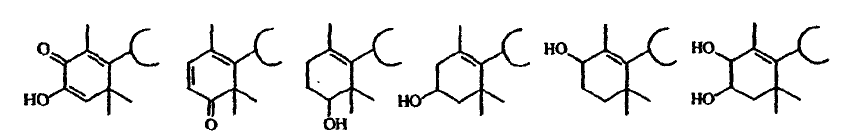

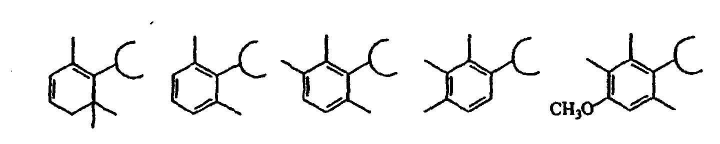

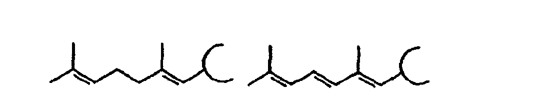

10. Procédé selon l'une quelconque des revendications 8 et 9, **caractérisé en ce qu'**il est réalisé en présence d'une base choisie de préférence dans le groupe constitué par les amidures, les hydrures et les alcoolates alcalins.

11. Procédé selon la revendication 10, **caractérisé en ce que** la base est le diisopropylamidure de lithium éventuellement associé au *N,N,N',N'*-tétraméthyléthylènediamine ou le 1,1,1,3,3,3-hexaméthyldisilazane.

12. Procédé selon l'une quelconque des revendications 8 à 11, **caractérisé en ce que** la réaction est réalisée dans un solvant choisi notamment dans le groupe constitué par le 1,2-diméthoxyéthane (DME), le *tert*-butyl-méthyléther, le tétrahydrofurane (THF), l'éther et leurs mélanges.

13. Procédé selon l'une quelconque des revendications 9 à 12, **caractérisé en ce que** l'on fait réagir l'énolate de la β-ionone de formule (5-a) 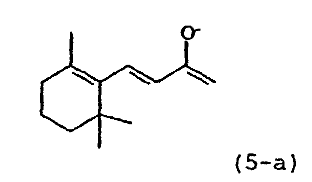 avec le composé de formule (1-j) 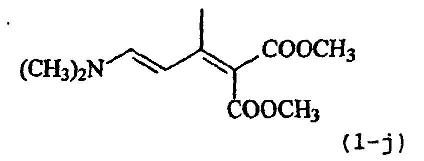 pour obtenir un composé de formule (6-a), sous la forme d'un mélange de 2 isomères 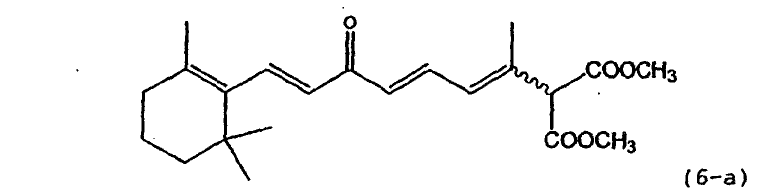 que l'on traite par un dérivé organométallique, halogénure de méthylmagnésium ou méthyllithium, pour obtenir un composé de formule (7-a) sous la forme de 2 isomères, 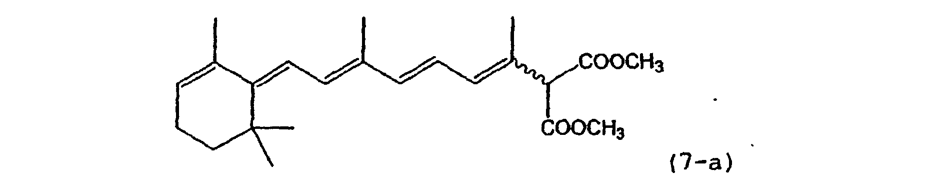 composé que l'on saponifie, pour obtenir l'acide rétinoïque *tout trans* de formule (8)

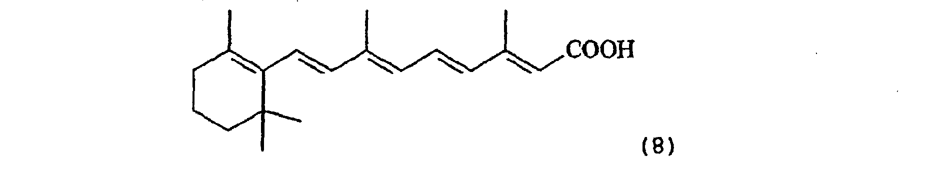

14. Procédé de synthèse de la vitamine A à partir de l'acide rétinoïque, **caractérisé en ce que** l'acide rétinoïque est préparé par un procédé selon l'une quelconque des revendications 8 à 13.

15. Utilisation des composés de formule (1) selon l'une quelconque des revendications 1 à 3 comme intermédiaires dans la synthèse des rétinoïdes et des carotinoïdes, notamment dans la synthèse des acides rétinoïques et de la vitamine A.

16. Utilisation selon la revendication 15, **caractérisée en ce que** l'on utilise le composé de formule (1-j) ou son analogue ester éthylique.

17. Utilisation selon l'une quelconque des revendications 15 et 16, **caractérisée en ce que** le rétinoïde est choisi dans le groupe constitué par l'acide rétinoïque, la vitamine A (rétinol), le rétinal, le rétinonitrile et l'étrétinate.

## Patentansprüche

1. Verbindungen der Formel (1) in der 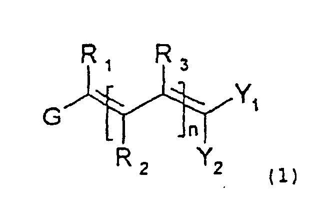
- G eine Gruppe -NR₄R₅ darstellt, worin R₄ und R₅, identisch oder verschieden, jeweils eine lineare oder verzweigte Alkylgruppe mit 1 bis 5 Kohlenstoffatomen oder eine Cycloalkylgruppe mit 3 bis 7 Kohlenstoffatomen sind oder R₄ und R₅ einen Cyclus mit dem Stickstoffatom bilden, das sie trägt,
- R₁ entweder ein Wasserstoffatom oder eine lineare oder verzweigte Alkylgruppe mit 1 bis 5 Kohlenstoffatomen darstellt,
- n eine ganze Zahl zwischen 1 und 2 einschließlich ist,
- R₂ und R₃ einen Substituenten darstellen, der in jeder der ungesättigten Einheiten gleichartig oder verschieden ist, wobei die aufeinanderfolgenden ungesättigten Einheiten darüber hinaus identisch oder verschieden sein können, wobei R₂ und R₃ aus der Gruppe ausgewählt sind, die Wasserstoff und lineare oder verzweigte Alkylgruppen mit 1 bis 5 Kohlenstoffatomen umfasst, und mit der Bedingung, dass mindestens einer der Substituenten R₁, R₂ und R₃ von H verschieden ist, und
- Y₁ und Y₂ jeweils eine Gruppe -COOR₇ darstellen, worin R₇ eine Alkylgruppe mit 1 bis 5 Kohlenstoffatomen ist.

2. Verbindungen nach Anspruch 1, **dadurch gekennzeichnet, dass**:
- G die Gruppe -N(CH₃)₂, -N(C₂H₅)₂ oder N-Pyrrolidin darstellt,
- n eine ganze Zahl gleich 1 oder 2 ist,
- R₁, R₂ und R₃ unabhängig voneinander ein Wasserstoffatom oder eine Methylgruppe darstellen, wobei die aufeinanderfolgenden ungesättigten Einheiten darüber hinaus identisch oder verschieden sein können, mit der Bedingung, dass mindestens einer der Substituenten R₁, R₂ und R₃ von H verschieden ist, und
- Y₁ und Y₂ jeweils eine Gruppe -COOCH₃ oder eine Gruppe -COOC₂H₅ darstellen.

3. Verbindungen nach irgendeinem der Ansprüche 1 und 2, **dadurch gekennzeichnet, dass** sie aus der Gruppe ausgewählt sind, die besteht aus: 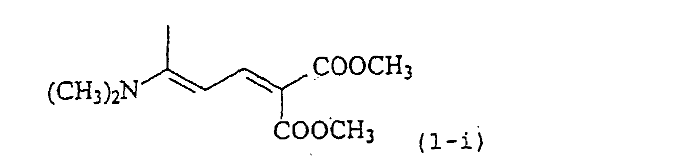 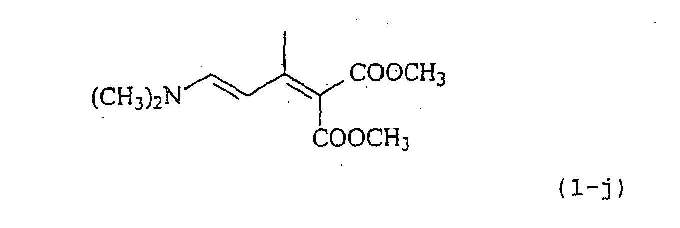 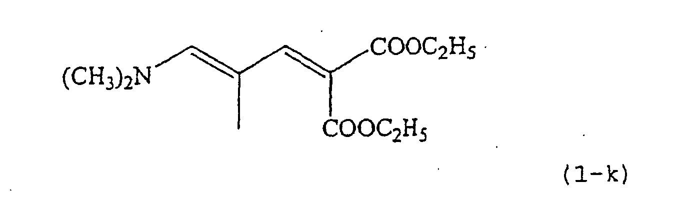 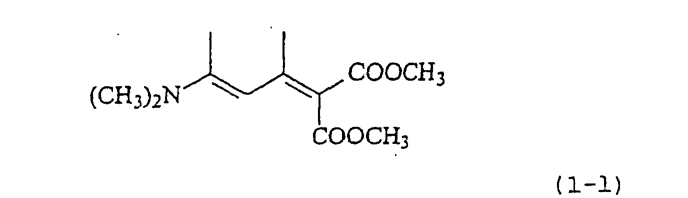 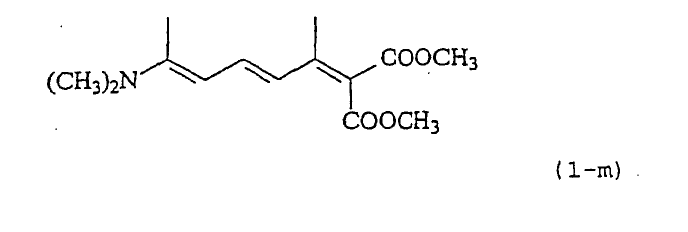 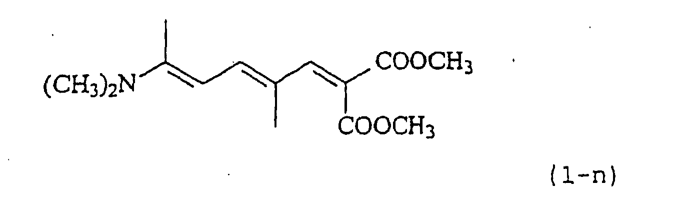 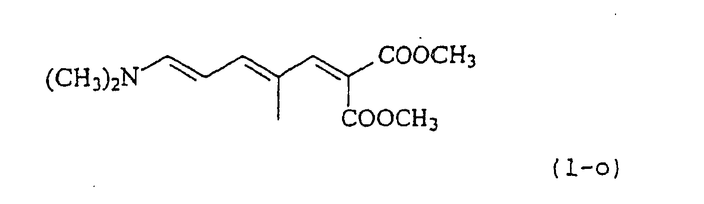 und den analogen Ethylestern der Verbindungen (1-i) bis (1-j) und (1-I) bis (1-o).

4. Herstellung der Verbindung (1-j) nach Anspruch 3, **dadurch gekennzeichnet, dass** man Methylisopropylidenmalonat der Formel (3-a) 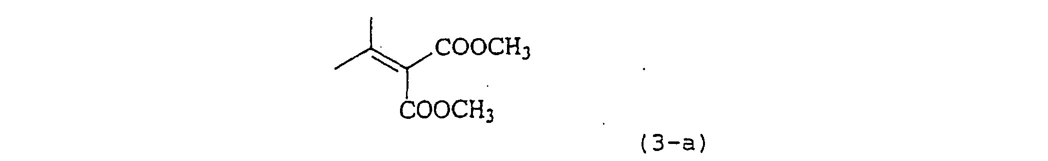 mit einer geschützten Form von N,N-Dimethylformamid, insbesondere DMFDMA der Formel (2-a) 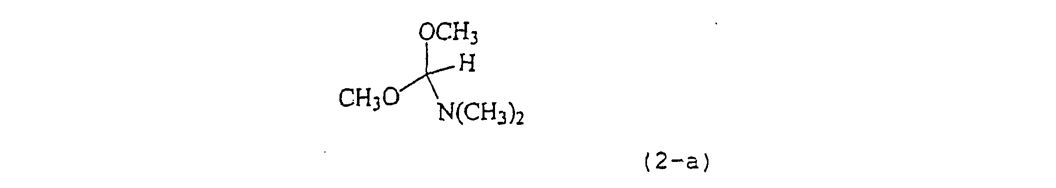 reagieren lässt.

5. Verfahren zur Synthese der Verbindungen 1-i, 1-l, 1-m und 1-n nach Anspruch 3, **dadurch gekennzeichnet, dass** man die Verbindung der Formel (3) 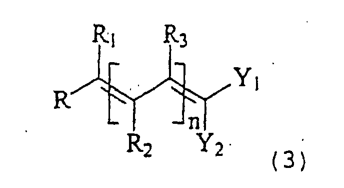 in der R₁, R₂ und R₃, Y₁ und Y₂ wie vorstehend definiert sind und R eine entsprechende Alkylgruppe (mit 1 oder 2 Kohlenstoffatomen) darstellt, mit der Verbindung der Formel (2-b) 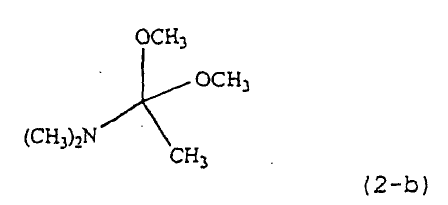 reagieren lässt.

6. Verfahren zur Synthese einer Verbindung der Formel (1) nach irgendeinem der Ansprüche 1 bis 3, die (n+1) Einheiten umfasst, ausgehend von einer Verbindung der Formel (1), die n Einheiten umfasst, **dadurch gekennzeichnet, dass** man in einem Lösungsmittel eine organometallische Verbindung mit einer Verbindung der Formel (1), die n Einheiten umfasst, reagieren lässt, um eine Verbindung der Formel (3) zu erhalten, die man mit einem N,N-Dimethylformamid- oder Acetamid-Derivat, vorzugsweise mit einer Verbindung der Formel (2-a) oder (2-b), behandelt.

7. Verfahren zur Synthese einer Verbindung der Formel (1), die n+2 Einheiten umfasst, ausgehend von einer Verbindung (1), die n Einheiten umfasst, **dadurch gekennzeichnet, dass** man in einem Lösungsmittel eine Verbindung der Formel (1), die n Einheiten umfasst, mit dem Enolat eines Ketons (vorzugsweise Aceton oder Butanon) reagieren lässt, wobei das Enolat durch eine Base erzeugt wird, dann eine organometallische Verbindung dazu gibt, vorzugsweise ein Alkyl- oder Arylmagnesiumhalogenid oder ein Alkyl- oder Aryllithium, und die erhaltene Verbindung der Formel (3) mit einem N,N-Dimethylformamid- oder Acetamid-Derivat, vorzugsweise mit einer Verbindung der Formel (2-a) oder (2-b), behandelt.

8. Verfahren zur Synthese von Retinoiden, **dadurch gekennzeichnet, dass** man als Zwischenprodukt eine Verbindung der Formel (1) nach irgendeinem der Ansprüche 1 bis 3 verwendet.

9. Verfahren nach Anspruch 8, **dadurch gekennzeichnet, dass** es mindestens einen Schritt umfasst, der darin besteht, eine Verbindung der Formel (1) mit einer Verbindung der Formel (5) 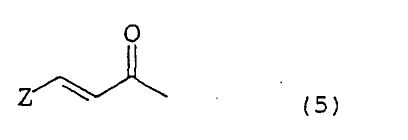 reagieren zu lassen, in welcher Z aus der Gruppe ausgewählt ist, welche die folgenden Reste umfasst:

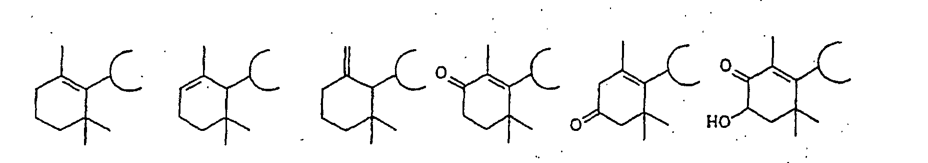

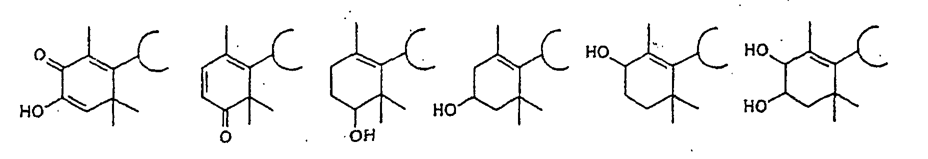

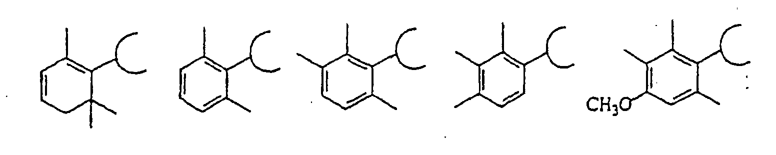

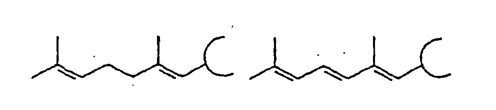

10. Verfahren nach irgendeinem der Ansprüche 8 und 9, **dadurch gekennzeichnet, dass** es in Anwesenheit einer Base durchgeführt wird, die vorzugsweise aus der Gruppe ausgewählt ist, die aus Alkaliamiden, - hydriden und -alkoholaten besteht.

11. Verfahren nach Anspruch 10, **dadurch gekennzeichnet, dass** die Base Lithiumdiisopropylamid ist, gegebenenfalls in Verbindung mit N,N,N',N'-Tetramethylethylendiamin oder 1,1,1,3,3,3-Hexamethyldisilazan.

12. Verfahren nach irgendeinem der Ansprüche 8 bis 11, **dadurch gekennzeichnet, dass** die Reaktion in einem Lösungsmittel durchgeführt wird, das insbesondere aus der Gruppe ausgewählt ist, die aus 1,2-Dimethoxyethan (DME), tert-Butylmethylether, Tetrahydrofuran (THF), Ether und deren Mischungen besteht.

13. Verfahren nach irgendeinem der Ansprüche 9 bis 12, **dadurch gekennzeichnet, dass** man β-lononenolat der Formel (5-a) 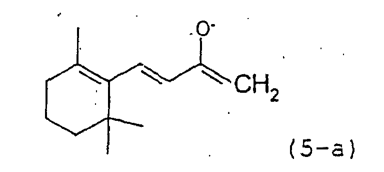 mit der Verbindung der Formel (1-j) 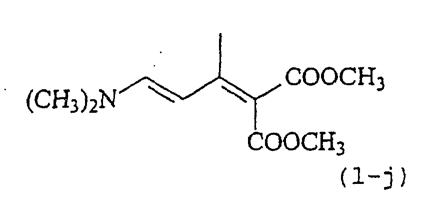 reagieren lässt, um eine Verbindung der Formel (6-a) in Form einer Mischung von 2 Isomeren zu erhalten, 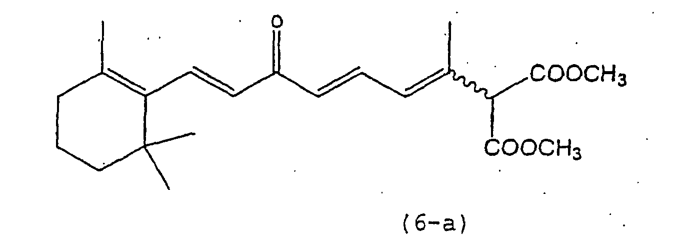 welche man mit einem organometallischen Derivat, Methylmagnesiumhalogenid oder Methyllithium, behandelt, um eine Verbindung der Formel (7-a) in Form von 2 Isomeren zu erhalten, 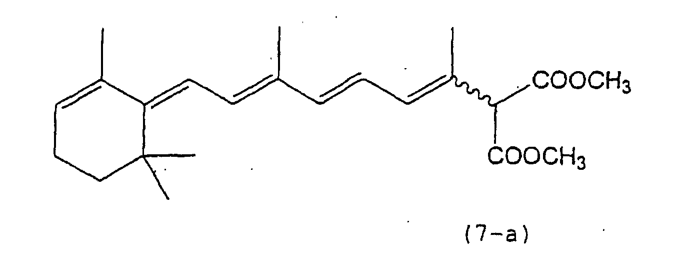 dann die Verbindung verseift, um all-trans-Retinoesäure der Formel (8) zu erhalten.

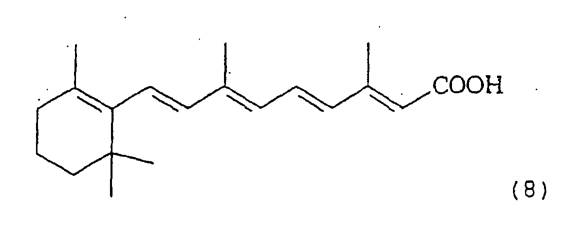

14. Verfahren zur Synthese von Vitamin A ausgehend von Retinoesäure, **dadurch gekennzeichnet, dass** die Retinoesäure durch ein Verfahren nach irgendeinem der Ansprüche 8 bis 13 hergestellt wird.

15. Verwendung der Verbindungen der Formel (1) nach irgendeinem der Ansprüche 1 bis 3 als Zwischenprodukte bei der Synthese von Retinoiden und Carotinoiden, insbesondere bei der Synthese von Retinoesäuren und von Vitamin A.

16. Verwendung nach Anspruch 15, **dadurch gekennzeichnet, dass** man die Verbindung der Formel (1-j) oder ihr Ethylester-Analogon verwendet.

17. Verwendung nach irgendeinem der Ansprüche 15 und 16, **dadurch gekennzeichnet, dass** das Retinoid aus der Gruppe ausgewählt ist, die aus Retinoesäure, Vitamin A (Retinol), Retinal, Retinonitril und Etretinat besteht.

## Claims

1. Compounds of formula (1) 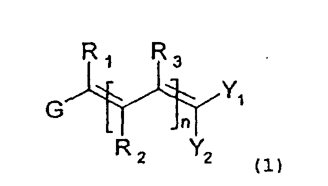 in which
- G represents a group -NR₄R₅ where R₄ and R₅, which are identical or different, are each a linear or branched alkyl group of 1 to 5 carbon atoms, or a cycloalkyl group of 3 to 7 carbon atoms, or R₄ and R₅ form a ring with the nitrogen atom carrying them,
- R₁ represents either a hydrogen atom or a linear or branched alkyl group of 1 to 5 carbon atoms,
- n is an integer between 1 and 2,
- R₂ and R₃ represent a similar or different substituent in each of the unsaturated units, it being possible moreover for said successive unsaturated units to be identical or different, R₂ and R₃ being chosen from the group comprising hydrogen and the linear or branched alkyl groups of 1 to 5 carbon atoms, and provided that at least one of the substituents R₁, R₂ and R₃ is different from H, and
Y₁ and Y₂ each represent a group -COOR₇ where R₇ is an alkyl group of 1 to 5 carbon atoms.

2. Compounds according to Claim 1, **characterized in that**:
- G represents the group -N(CH₃)₂, -N(C₂H₅)₂ or N-pyrrolidine,
- n is an integer equal to 1 or 2,
- R₁, R₂ and R₃ represent, independently of each other, a hydrogen atom or a methyl group, it being possible moreover for the successive unsaturated units to be identical or different, provided that at least one of the substituents R₁, R₂ and R₃ is different from H, and - Y₁ and Y₂ each represent a group -COOCH₃ or a group -COOC₂H₅.

3. Compounds according to either of Claims 1 and 2, **characterized in that** they are chosen from the group consisting of: 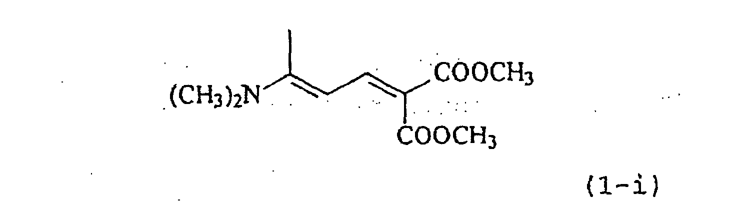 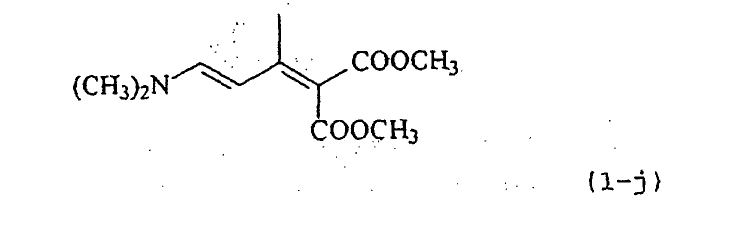 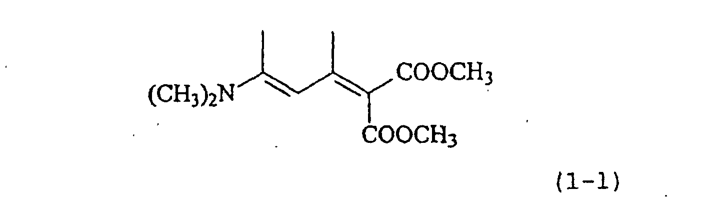 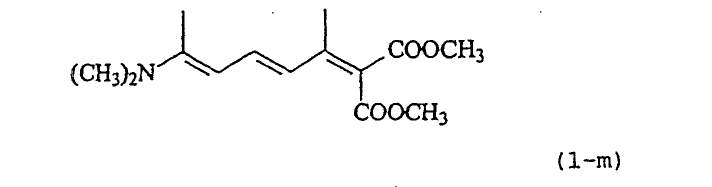 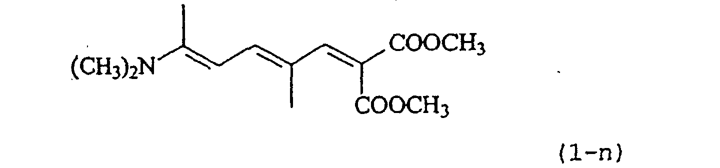 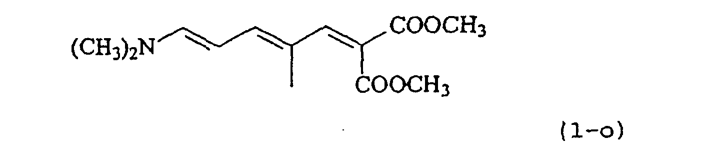 and the ethyl ester analogues of the compounds (1-i) to (1-j) and (1-1) to (1-o).

4. Preparation of the compound (1-j) according to Claim 3, **characterized in that** methyl isopropylidenemalonate of formula (3-a) 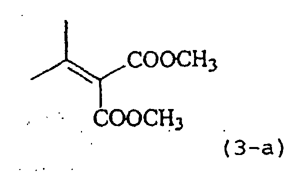 is reacted with a protected form of *N,N*-dimethylformamide, in particular DMFDMA of formula (2-a)

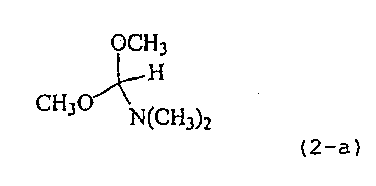

5. Method for synthesizing the compounds 1-i, 1-l, 1-m and 1-n according to Claim 3, **characterized in that** the compound of formula (3) 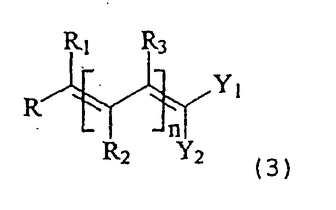 in which R₁, R₂ and R₃, Y₁ and Y₂ are as defined above and R represents a corresponding alkyl group (of 1 or 2 carbon atoms), is reacted with the compound of formula (2-b)

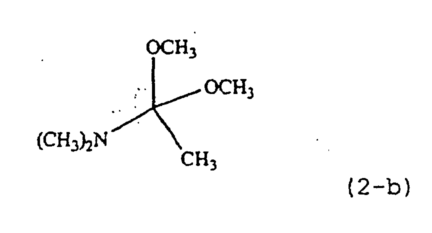

6. Method for synthesizing a compound of formula (1) according to any one of Claims 1 to 3, comprising n+1 units, from a compound of formula (1) comprising n units, **characterized in that** an organometallic is reacted, in a solvent, with a compound of formula (1) comprising n units, in order to obtain a compound of formula (3) which is treated with an *N,N*-dimethylformamide or acetamide derivative, preferably with a compound of formula (2-a) or (2-b).

7. Method for synthesizing a compound of formula (1) comprising n+2 units from a compound (1) comprising n units, **characterized in that** a compound of formula (1) comprising n units is reacted, in a solvent, with the enolate of a ketone (preferably acetone or butanone), said enolate being generated by a base, and then an organometallic, preferably an alkyl- or arylmagnesium halide or an alkyl- or aryllithium, is added and the compound of formula (3) obtained is treated with an N,N-dimethylformamide or acetamide derivative, preferably with a compound of formula (2-a) or (2-b).

8. Method for synthesizing retinoids, **characterized in that** a compound of formula (1) according to any one of Claims 1 to 3 is used as an intermediate.

9. Method according to Claim 8, **characterized in that** it comprises at least one step consisting in reacting a compound of formula (1) with a compound of formula (5) 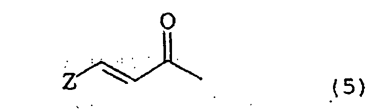 in which Z is chosen from the group comprising the following radicals:

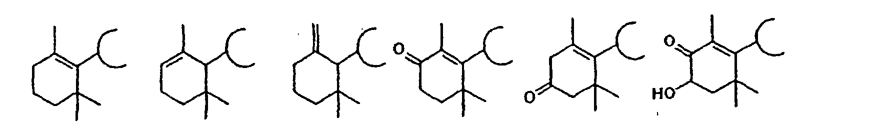

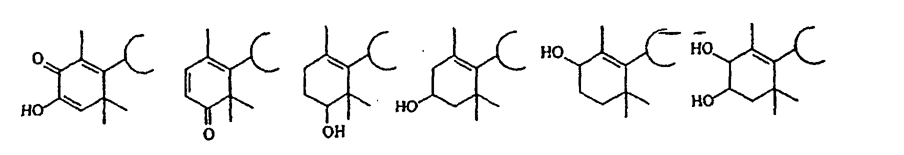

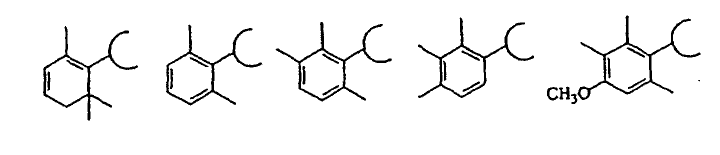

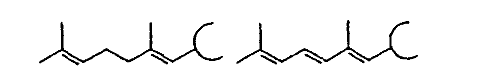

10. Method according to either of Claims 8 and 9, **characterized in that** it is carried out in the presence of a base, preferably chosen from the group consisting of alkali metal amides, hydrides and alcoholates.

11. Method according to Claim 10, **characterized in that** the base is lithium diisopropylamide, optionally combined with *N,N,N',N'*-tetramethylethylenediamine or 1,1,1,3,3,3-hexamethyldisilazane.

12. Method according to any one of Claims 8 to 11, **characterized in that** the reaction is carried out in a solvent chosen in particular from the group consisting of 1,2-dimethoxyethane (DME), tert-butyl methyl ether, tetrahydrofuran (THF), ether and mixtures thereof.

13. Method according to any one of Claims 9 to 12, **characterized in that** the β-ionone enolate of formula (5-a) 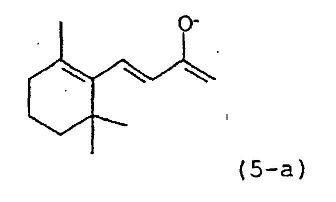 is reacted with the compound of formula (1-j) 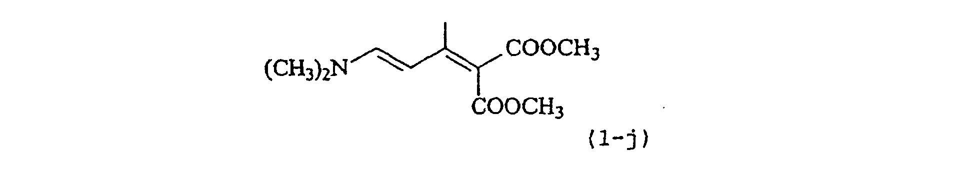 to give a compound of formula (6-a), in the form of a mixture of 2 isomers 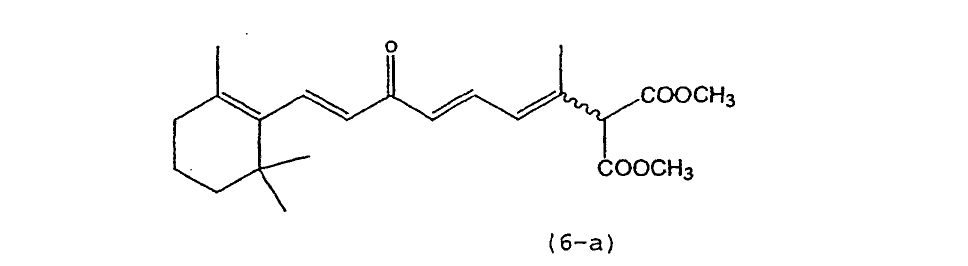 which is treated with an organometallic derivative, methylmagnesium or methyllithium halide, to give a compound of formula (7-a) in the form of 2 isomers, 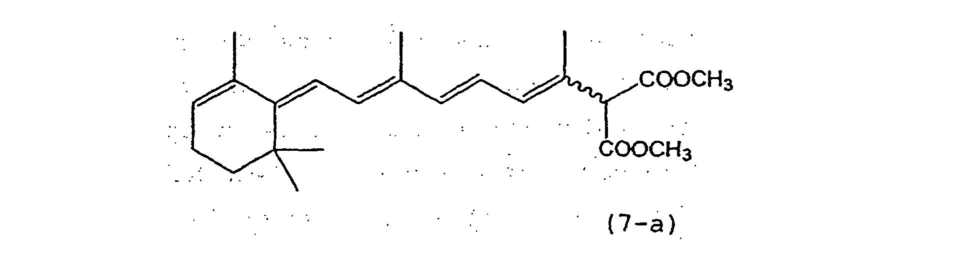 which compound is saponified, to give the *all-trans*-retinoic acid of formula (8)

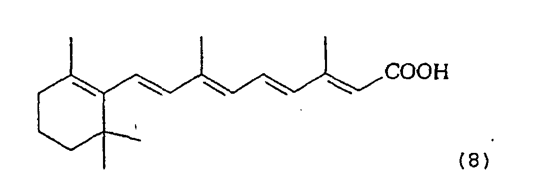

14. Method for synthesizing vitamin A from retinoic acid, **characterized in that** the retinoic acid is prepared by a method according to any one of Claims 8 to 13.

15. Use of the compounds of formula (1) according to any one of Claims 1 to 3 as intermediates in the synthesis of retinoids and carotenoids, in particular in the synthesis of retinoic acids and vitamin A.

16. Use according to Claim 15, **characterized in that** the compound of formula (1-j) or its ethyl ester analogue is used.

17. Use according to either of Claims 15 and 16, **characterized in that** the retinoid is chosen from the group consisting of retinoic acid, vitamin A (retinol), retinal, retinonitrile and etretinate.
